# EUROPEAN PATENT SPECIFICATION

(11) **EP 4 188 208 B1**
(45) Date of publication and mention of the grant of the patent: **08.04.2026**
(21) Application number: 21749490.5
(22) Date of filing: 12.07.2021
(51) Int. Cl.: A61B 5/0245, A61B 5/282, A61B 5/349, A61B 5/00, A61N 1/362, A61B 5/363, A61B 5/366

(54) **PATIENT SCREENING AND ECG BELT FOR BRADY THERAPY TUNING**
PATIENTENSCREENING UND EKG-GÜRTEL ZUR BRADYTHERAPIEABSTIMMUNG
CRIBLAGE DE PATIENT ET CEINTURE D'ECG POUR MODULATION DE THÉRAPIE DE BRADYCARDIE

(30) Priority: 30.07.2020 US 202063059135 P; 06.07.2021 US 202117368260
(43) Date of publication of application: 07.06.2023
(73) Proprietor: Medtronic, Inc., Minneapolis, Minnesota 55432 (US)
(72) Inventor: KLEPFER, Ruth N., Minneapolis, Minnesota 55432 (US); JUSTEN, Manfred, Minneapolis, Minnesota 55432 (US); GHOSH, Subham, Minneapolis, Minnesota 55432 (US); GILLBERG, Jeffrey M., Minneapolis, Minnesota 55432 (US)
(74) Representative: Zimmermann & Partner Patentanwälte mbB
(86) International application number: PCT/US2021/041208
(87) International publication number: WO 2022/026162

(56) References cited:
- US-A1- 2017 246 460
- US-A1- 2017 246 461
- US-A1- 2017 303 840
- US-A1- 2019 269 926
- US-A1- 2019 366 106
- YAHYA S. AL HEBAISHI ET AL: "Predictors of Cardiac Resynchronization Therapy Response: The Pivotal Role of Electrocardiogram", THE SCIENTIFIC WORLD JOURNAL, vol. 2013, 1 January 2013 (2013-01-01), pages 1 - 6, XP055648226, DOI: 10.1155/2013/837086
- SIENIEWICZ BENJAMIN J ET AL: "Understanding non-response to cardiac resynchronisation therapy: common problems and potential solutions", HEART FAILURE REVIEWS, KLUWER ACADEMIC PUBLISHERS, DORDRECHT, NL, vol. 24, no. 1, 24 August 2018 (2018-08-24), pages 41 - 54, XP036665004, ISSN: 1382-4147, [retrieved on 20180824], DOI: 10.1007/S10741-018-9734-8
- RICKARD JOHN ET AL: "The ECG Belt for CRT response trial: Design and clinical protocol", PACE - PACING AND CLINICAL ELECTROPHYSIOLOGY., vol. 43, no. 10, 14 June 2020 (2020-06-14), US, pages 1063 - 1071, XP055845558, ISSN: 0147-8389, Retrieved from the Internet <URL:https://onlinelibrary.wiley.com/doi/full-xml/10.1111/pace.13985> [retrieved on 20210929], DOI: 10.1111/pace.13985

## Description

The disclosure herein relates to systems and methods for use in the determining whether a patient is a candidate for a cardiac resynchronization device (CRT) and whether bradycardia pacing therapy is effective.

Implantable medical devices (IMDs), such as implantable pacemakers, cardioverters, defibrillators, or pacemaker-cardioverter-defibrillators, provide therapeutic electrical stimulation to the heart. IMDs may provide pacing to address bradycardia, or pacing or shocks in order to terminate tachyarrhythmia, such as tachycardia or fibrillation. In some cases, the medical device may sense intrinsic depolarizations of the heart, detect arrhythmia based on the intrinsic depolarizations (or absence thereof), and control delivery of electrical stimulation to the heart if arrhythmia is detected based on the intrinsic depolarizations.

IMDs may also provide cardiac resynchronization therapy (CRT), which is a form of pacing. CRT involves the delivery of pacing to the left ventricle, or both the left and right ventricles. The timing and location of the delivery of pacing pulses to the ventricle(s) may be selected to improve the coordination and efficiency of ventricular contraction.

Systems for implanting medical devices may include workstations or other equipment in addition to the implantable medical device itself. In some cases, these other pieces of equipment assist the physician or other technician with placing the intracardiac leads at particular locations on the heart. In some cases, the equipment provides information to the physician about the electrical activity of the heart and the location of the intracardiac lead. The equipment may perform similar functions as the medical device, including delivering electrical stimulation to the heart and sensing the depolarizations of the heart. In some cases, the equipment may include equipment for obtaining an electrocardiogram (ECG) via electrodes on the surface, or skin, of the patient. More specifically, the patient may have a plurality of electrodes on an ECG belt or vest that surrounds the torso of the patient. After the belt or vest has been secured to the torso, a physician can perform a series of tests to evaluate a patient's cardiac response. The evaluation process can include detection of a baseline rhythm in which no electrical stimuli is delivered to cardiac tissue and another rhythm after electrical stimuli is delivered to the cardiac tissue.

The ECG electrodes placed on the body surface of the patient may be used for various therapeutic purposes (e.g., cardiac resynchronization therapy) including optimizing lead location, pacing parameters, etc. based on one or more metrics derived from the signals captured by the ECG electrodes. For example, electrical heterogeneity information may be derived from electrical activation times computed from multiple electrodes on the body surface.

US 2017/246460 A1 describes methods and systems of evaluating cardiac pacing in candidate patients for cardiac resynchronization therapy and cardiac resynchronization therapy patients.

### SUMMARY

The exemplary systems and methods described herein may be configured to assist users (e.g., physicians) in deciding on and/or configuring cardiac therapy (e.g., selecting which patients receive a particular cardiac therapy, or configuring a cardiac therapy being performed on a patient during and/ or after implantation of cardiac therapy apparatus). The systems and methods may be described as being noninvasive. For example, the systems and methods may not need implantable devices such as leads, probes, sensors, catheters, etc. to evaluate and configure the cardiac therapy. Instead, the systems and methods may use electrical measurements taken noninvasively using, e.g., a plurality of external electrodes attached to the skin of a patient about the patient's torso.

One embodiment involves a system for use in cardiac evaluation comprising an electrode apparatus comprising a plurality of external electrodes to be disposed proximate a patient's skin. A computing apparatus comprising processing circuitry is operably coupled to the electrode apparatus. The computing apparatus is configured to monitor cardiac electrical activity from tissue of the patient using the plurality of external electrodes. One or more cardiac metrics of the patient are generated based on the monitored electrical activity. It is determined whether the patient is a candidate for a cardiac resynchronization therapy (CRT) device based on a first global dyssynchrony metric using the one or more cardiac metrics if the patient has a right bundle branch block. It is determined whether the patient is a candidate for a cardiac resynchronization therapy (CRT) device based on a second global dyssynchrony metric using the one or more cardiac metrics if the patient does not have a right bundle branch block.

One embodiment involves a system for use in cardiac evaluation comprising an electrode apparatus comprising a plurality of external electrodes to be disposed proximate a patient's skin. A computing apparatus comprising processing circuitry is operably coupled to the electrode apparatus. The computing apparatus is configured to monitor cardiac electrical activity from tissue of the patient using the plurality of external electrodes during delivery of bradycardia pacing to the patient's heart. Electrical heterogeneity information (EHI) is generated based on the monitored cardiac electrical activity during delivery of bradycardia pacing to the patient's heart. It is determined whether the bradycardia pacing therapy is effective based on the generated EHI, wherein the EHI is reflective of normalization of conduction for a population of patients during bradycardia pacing.

One embodiment involves a method for use in cardiac evaluation comprising monitoring cardiac electrical activity from tissue of the patient using the plurality of external electrodes during delivery of bradycardia pacing to the patient's heart. Electrical heterogeneity information (EHI) is generated based on the monitored cardiac electrical activity during delivery of bradycardia pacing to the patient's heart. It is determined whether the bradycardia pacing therapy is effective based on the generated EHI, wherein the EHI is reflective of normalization of conduction for a population of patients during bradycardia pacing.

Traditional ECG-based metrics like QRS duration/morphology have been used as guidelines for CRT but are often not as sensitive or specific. The result is that patients in class II indication (non-left bundle or left bundle with narrower QRS.

The ECG belt is a multi-electrode body-surface mapping system that provides metrics of electrical dyssynchrony. Changes in electrical dyssynchrony from intrinsic to CRT/LV pacing has been shown to be useful for titrating LV lead location, optimizing device programming parameters (vectors, timing, etc). The use of an ECG belt in a brady population for feedback on choice of pacing therapy (e.g. His bundle/LBB-area pacing vs traditional RV lead or a combination of both, different locations of RV lead) is described herein

### BRIEF DESCRIPTION OF THE DRAWINGS

FIG. 1 is a diagram of an exemplary system including electrode apparatus, display apparatus, and computing apparatus.
FIGS. 2-3 are diagrams of exemplary external electrode apparatus for measuring torso-surface potentials.
FIGS. 4-5 show exemplary methods for determining whether a patient is a candidate for a cardiac resynchronization device (CRT).
FIGS. 6-8 illustrate graphs of intrinsic SDAT vs intrinsic LVAT for 115 patients.
FIGS. 9-10 show exemplary methods for determining whether bradycardia pacing therapy is effective.
FIG. 11 is a diagram of an illustrative system including an illustrative implantable medical device (IMD).
FIG. 12A is a diagram of the illustrative IMD of FIG. 11.
FIG. 12B is a diagram of an enlarged view of a distal end of the electrical lead disposed in the left ventricle of FIG. 12A.
FIG. 13 is a conceptual diagram of an illustrative cardiac therapy system including an intracardiac medical device implanted in a patient's heart and a separate medical device positioned outside of the patient's heart.
FIG. 14 is an enlarged conceptual diagram of the intracardiac medical device of FIG. 13 and anatomical structures of the patient's heart.
FIG. 15 is a conceptual diagram of a map of a patient's heart in a standard 17 segment view of the left ventricle showing various electrode implantation locations for use with the illustrative systems and devices described herein.
FIG. 16A is a block diagram of an illustrative IMD.
FIG. 16B is another block diagram of an illustrative IMD.

### DETAILED DESCRIPTION OF EXEMPLARY EMBODIMENTS

In the following detailed description of illustrative embodiments, reference is made to the accompanying figures of the drawing which form a part hereof, and in which are shown, by way of illustration, specific embodiments which may be practiced. It is to be understood that other embodiments may be utilized and structural changes may be made without departing from (e.g., still falling within) the scope of the disclosure presented hereby.

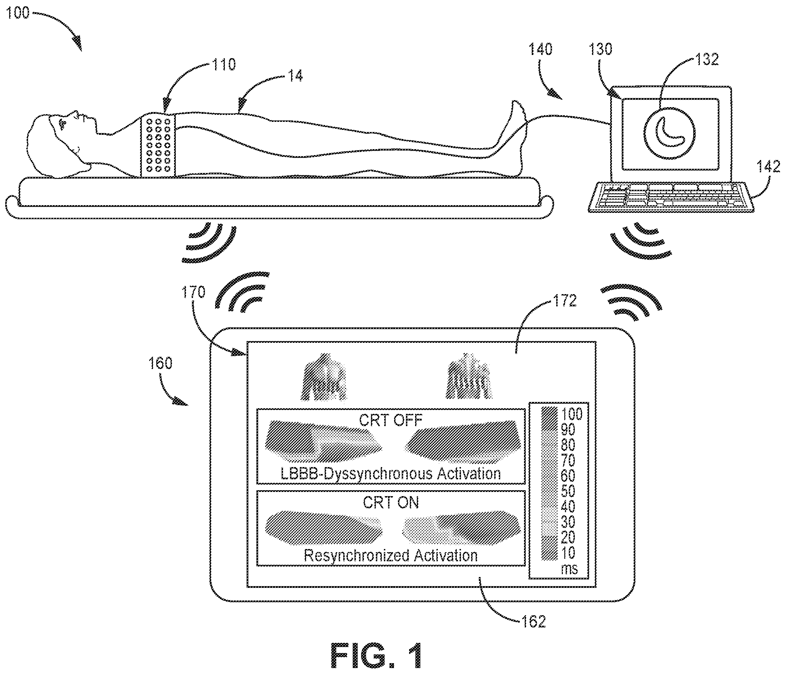

Illustrative systems and methods shall be described with reference to FIGS. 1-12. It will be apparent to one skilled in the art that elements or processes from one embodiment may be used in combination with elements or processes of the other embodiments, and that the possible embodiments of such systems, methods, and devices using combinations of features set forth herein is not limited to the specific embodiments shown in the Figures and/or described herein. Further, it will be recognized that the embodiments described herein may include many elements that are not necessarily shown to scale. Still further, it will be recognized that timing of the processes and the size and shape of various elements herein may be modified but still fall within the scope of the present disclosure, although certain timings, one or more shapes and/or sizes, or types of elements, may be advantageous over others.

A plurality of electrocardiogram (ECG) signals (e.g., torso-surface potentials) may be measured, or monitored, using a plurality of external electrodes positioned about the surface, or skin, of a patient. The ECG signals may be used to evaluate and configure cardiac therapy such as, e.g., cardiac therapy provide by an implantable medical device performing cardiac resynchronization therapy (CRT). As described herein, the ECG signals may be gathered or obtained noninvasively since, e.g., implantable electrodes may not be used to measure the ECG signals. Further, the ECG signals may be used to determine cardiac electrical activation times, which may be used to generate various metrics (e.g., electrical heterogeneity information) that may be used by a user (e.g., physician) to optimize one or more settings, or parameters, of cardiac therapy (e.g., pacing therapy) such as CRT.

Various illustrative systems, methods, and graphical user interfaces may be configured to use electrode apparatus including external electrodes, display apparatus, and computing apparatus to noninvasively assist a user (e.g., a physician) in the evaluation of cardiac health and/or the configuration (e.g., optimization) of cardiac therapy. An illustrative system 100 including electrode apparatus 110, computing apparatus 140, and a remote computing device 160 is depicted in FIG. 1.

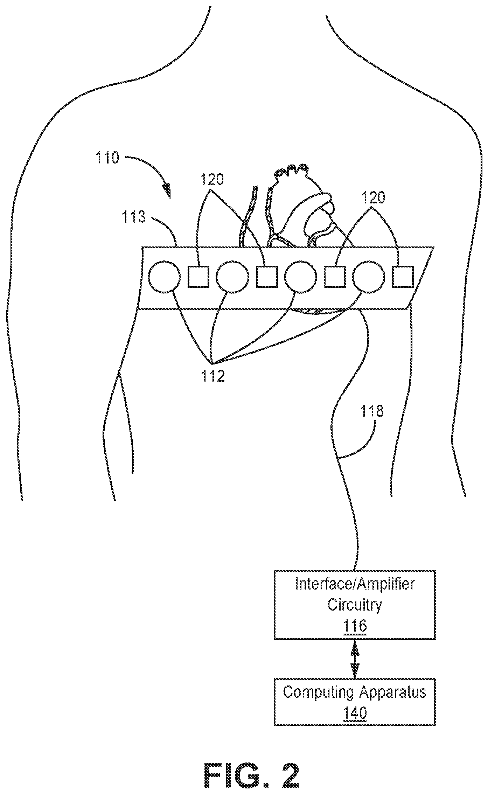

The electrode apparatus 110 as shown includes a plurality of electrodes incorporated, or included, within a band wrapped around the chest, or torso, of a patient 14. The electrode apparatus 110 is operatively coupled to the computing apparatus 140 (e.g., through one or wired electrical connections, wirelessly, etc.) to provide electrical signals from each of the electrodes to the computing apparatus 140 for analysis, evaluation, etc. Illustrative electrode apparatus may be described in U.S. Patent No. 9,320,446 entitled "Bioelectric Sensor Device and Methods" filed March 27, 2014 and issued on March 26, 2016. Further, illustrative electrode apparatus 110 will be described in more detail in reference to FIGS. 2-3.

Although not described herein, the illustrative system 100 may further include imaging apparatus. The imaging apparatus may be any type of imaging apparatus configured to image, or provide images of, at least a portion of the patient in a noninvasive manner. For example, the imaging apparatus may not use any components or parts that may be located within the patient to provide images of the patient except noninvasive tools such as contrast solution. It is to be understood that the illustrative systems, methods, and interfaces described herein may further use imaging apparatus to provide noninvasive assistance to a user (e.g., a physician) to locate, or place, one or more pacing electrodes proximate the patient's heart in conjunction with the configuration of cardiac therapy.

For example, the illustrative systems and methods may provide image guided navigation that may be used to navigate leads including electrodes, leadless electrodes, wireless electrodes, catheters, etc., within the patient's body while also providing noninvasive cardiac therapy configuration including determining an effective, or optimal, pre-excitation intervals such as A-V and V-V intervals, etc. Illustrative systems and methods that use imaging apparatus and/or electrode apparatus may be described in U.S. Pat. App. Pub. No. 2014/0371832 to Ghosh published on December 18, 2014, U.S. Pat. App. Pub. No. 2014/0371833 to Ghosh et al. published on December 18, 2014, U.S. Pat. App. Pub. No. 2014/0323892 to Ghosh et al. published on October 30, 2014, U.S. Pat. App. Pub. No. 2014/0323882 to Ghosh et al. published on October 20, 2014.

Illustrative imaging apparatus may be configured to capture x-ray images and/or any other alternative imaging modality. For example, the imaging apparatus may be configured to capture images, or image data, using isocentric fluoroscopy, bi-plane fluoroscopy, ultrasound, computed tomography (CT), multi-slice computed tomography (MSCT), magnetic resonance imaging (MRI), high frequency ultrasound (HIFU), optical coherence tomography (OCT), intravascular ultrasound (IVUS), two dimensional (2D) ultrasound, three dimensional (3D) ultrasound, four dimensional (4D) ultrasound, intraoperative CT, intraoperative MRI, etc. Further, it is to be understood that the imaging apparatus may be configured to capture a plurality of consecutive images (e.g., continuously) to provide video frame data. In other words, a plurality of images taken over time using the imaging apparatus may provide video frame, or motion picture, data. An exemplary system that employs ultrasound can be found in U.S. Pat. App. Pub. No. 2017/0303840 entitled NONINVASIVE ASSESSMENT OF CARDIAC RESYNCHRONIZATION THERAPY to Stadler et al. Additionally, the images may also be obtained and displayed in two, three, or four dimensions. In more advanced forms, four-dimensional surface rendering of the heart or other regions of the body may also be achieved by incorporating heart data or other soft tissue data from a map or from pre-operative image data captured by MRI, CT, or echocardiography modalities. Image datasets from hybrid modalities, such as positron emission tomography (PET) combined with CT, or single photon emission computer tomography (SPECT) combined with CT, could also provide functional image data superimposed onto anatomical data, e.g., to be used to navigate implantable apparatus to target locations within the heart or other areas of interest.

Systems and/or imaging apparatus that may be used in conjunction with the illustrative systems and method described herein are described in U.S. Pat. App. Pub. No. 2005/0008210 to Evron et al. published on January 13, 2005, U.S. Pat. App. Pub. No. 2006/0074285 to Zarkh et al. published on April 6, 2006, U.S. Pat. No. 8,731,642 to Zarkh et al. issued on May 20, 2014, U.S. Pat. No. 8,861,830 to Brada et al. issued on October 14, 2014, U.S. Pat. No. 6,980,675 to Evron et al. issued on December 27, 2005, U.S. Pat. No. 7,286,866 to Okerlund et al. issued on October 23, 2007, U.S. Pat. No. 7,308,297 to Reddy et al. issued on December 11, 2011, U.S. Pat. No. 7,308,299 to Burrell et al. issued on December 11, 2011, U.S. Pat. No. 7,321,677 to Evron et al. issued on January 22, 2008, U.S. Pat. No. 7,346,381 to Okerlund et al. issued on March 18, 2008, U.S. Pat. No. 7,454,248 to Burrell et al. issued on November 18, 2008, U.S. Pat. No. 7,499,743 to Vass et al. issued on March 3, 2009, U.S. Pat. No. 7,565,190 to Okerlund et al. issued on July 21, 2009, U.S. Pat. No. 7,587,074 to Zarkh et al. issued on September 8, 2009, U.S. Pat. No. 7,599,730 to Hunter et al. issued on October 6, 2009, U.S. Pat. No. 7,613,500 to Vass et al. issued on November 3, 2009, U.S. Pat. No. 7,742,629 to Zarkh et al. issued on June 22, 2010, U.S. Pat. No. 7,747,047 to Okerlund et al. issued on June 29, 2010, U.S. Pat. No. 7,778,685 to Evron et al. issued on August 17, 2010, U.S. Pat. No. 7,778,686 to Vass et al. issued on August 17, 2010, U.S. Pat. No. 7,813,785 to Okerlund et al. issued on October 12, 2010, U.S. Pat. No. 7,996,063 to Vass et al. issued on August 9, 2011, U.S. Pat. No. 8,060,185 to Hunter et al. issued on November 15, 2011, and U.S. Pat. No. 8,401,616 to Verard et al. issued on March 19, 2013.

The computing apparatus 140 and the remote computing device 160 may each include display apparatus 130, 170, respectively, that may be configured to display and analyze data such as, e.g., electrical signals (e.g., electrocardiogram data), electrical activation times, electrical heterogeneity information, etc. For example, one cardiac cycle, or one heartbeat, of a plurality of cardiac cycles, or heartbeats, represented by the electrical signals collected or monitored by the electrode apparatus 110 may be analyzed and evaluated for one or more metrics including activation times and electrical heterogeneity information that may be pertinent to the therapeutic nature of one or more parameters related to cardiac therapy such as, e.g., pacing parameters, lead location, etc. More specifically, for example, the QRS complex of a single cardiac cycle may be evaluated for one or more metrics such as, e.g., QRS onset, QRS offset, QRS peak, electrical heterogeneity information (EHI), electrical activation times referenced to earliest activation time, left ventricular or thoracic standard deviation of electrical activation times (LVED), standard deviation of activation times (SDAT), average left ventricular or thoracic surrogate electrical activation times (LVAT), QRS duration (e.g., interval between QRS onset to QRS offset), difference between average left surrogate and average right surrogate activation times, relative or absolute QRS morphology, difference between a higher percentile and a lower percentile of activation times (higher percentile may be 90%, 80%, 75%, 70%, etc. and lower percentile may be 10%, 15%, 20%, 25% and 30%, etc.), other statistical measures of central tendency (e.g., median or mode), dispersion (e.g., mean deviation, standard deviation, variance, interquartile deviations, range), etc. Further, each of the one or more metrics may be location specific. For example, some metrics may be computed from signals recorded, or monitored, from electrodes positioned about a selected area of the patient such as, e.g., the left side of the patient, the right side of the patient, etc.

In at least one embodiment, one or both of the computing apparatus 140 and the remote computing device 160 may be a server, a personal computer, a tablet computer, a mobile device, and a cellular telephone. The computing apparatus 140 may be configured to receive input from input apparatus 142 (e.g., a keyboard) and transmit output to the display apparatus 130, and the remote computing device 160 may be configured to receive input from input apparatus 162 (e.g., a touchscreen) and transmit output to the display apparatus 170. One or both of the computing apparatus 140 and the remote computing device 160 may include data storage that may allow for access to processing programs or routines and/or one or more other types of data, e.g., for analyzing a plurality of electrical signals captured by the electrode apparatus 110, for determining QRS onsets, QRS offsets, medians, modes, averages, peaks or maximum values, valleys or minimum values, for determining electrical activation times, for driving a graphical user interface configured to noninvasively assist a user in configuring one or more pacing parameters, or settings, such as, e.g., pacing rate, ventricular pacing rate, A-V interval, V-V interval, pacing pulse width, pacing vector, multipoint pacing vector (e.g., left ventricular vector quad lead), pacing voltage, pacing configuration (e.g., biventricular pacing, right ventricle only pacing, left ventricle only pacing, etc.), and arrhythmia detection and treatment, rate adaptive settings and performance, etc.

The computing apparatus 140 may be operatively coupled to the input apparatus 142 and the display apparatus 130 to, e.g., transmit data to and from each of the input apparatus 142 and the display apparatus 130, and the remote computing device 160 may be operatively coupled to the input apparatus 162 and the display apparatus 170 to, e.g., transmit data to and from each of the input apparatus 162 and the display apparatus 170. For example, the computing apparatus 140 and the remote computing device 160 may be electrically coupled to the input apparatus 142, 162 and the display apparatus 130, 170 using, e.g., analog electrical connections, digital electrical connections, wireless connections, bus-based connections, network-based connections, internet-based connections, etc. As described further herein, a user may provide input to the input apparatus 142, 162 to view and/or select one or more pieces of configuration information related to the cardiac therapy delivered by cardiac therapy apparatus such as, e.g., an implantable medical device.

Although as depicted the input apparatus 142 is a keyboard and the input apparatus 162 is a touchscreen, it is to be understood that the input apparatus 142, 162 may include any apparatus capable of providing input to the computing apparatus 140 and the computing device 160 to perform the functionality, methods, and/or logic described herein. For example, the input apparatus 142, 162 may include a keyboard, a mouse, a trackball, a touchscreen (e.g., capacitive touchscreen, a resistive touchscreen, a multi-touch touchscreen, etc.), etc. Likewise, the display apparatus 130, 170 may include any apparatus capable of displaying information to a user, such as a graphical user interface 132, 172 including electrode status information, graphical maps of electrical activation, a plurality of signals for the external electrodes over one or more heartbeats, QRS complexes, various cardiac therapy scenario selection regions, various rankings of cardiac therapy scenarios, various pacing parameters, electrical heterogeneity information (EHI), textual instructions, graphical depictions of anatomy of a human heart, images or graphical depictions of the patient's heart, graphical depictions of locations of one or more electrodes, graphical depictions of a human torso, images or graphical depictions of the patient's torso, graphical depictions or actual images of implanted electrodes and/or leads, etc. Further, the display apparatus 130, 170 may include a liquid crystal display, an organic light-emitting diode screen, a touchscreen, a cathode ray tube display, etc.

The processing programs or routines stored and/or executed by the computing apparatus 140 and the remote computing device 160 may include programs or routines for computational mathematics, matrix mathematics, decomposition algorithms, compression algorithms (e.g., data compression algorithms), calibration algorithms, image construction algorithms, signal processing algorithms (e.g., various filtering algorithms, Fourier transforms, fast Fourier transforms, etc.), standardization algorithms, comparison algorithms, vector mathematics, or any other processing used to implement one or more illustrative methods and/or processes described herein. Data stored and/or used by the computing apparatus 140 and the remote computing device 160 may include, for example, electrical signal/waveform data from the electrode apparatus 110 (e.g., a plurality of QRS complexes), electrical activation times from the electrode apparatus 110, cardiac sound/signal/waveform data from acoustic sensors, graphics (e.g., graphical elements, icons, buttons, windows, dialogs, pull-down menus, graphic areas, graphic regions, 3D graphics, etc.), graphical user interfaces, results from one or more processing programs or routines employed according to the disclosure herein (e.g., electrical signals, electrical heterogeneity information, etc.), or any other data that may be used for carrying out the one and/or more processes or methods described herein.

In one or more embodiments, the illustrative systems, methods, and interfaces may be implemented using one or more computer programs executed on programmable computers, such as computers that include, for example, processing capabilities, data storage (e.g., volatile or non-volatile memory and/or storage elements), input devices, and output devices. Program code and/or logic described herein may be applied to input data to perform functionality described herein and generate desired output information. The output information may be applied as input to one or more other devices and/or methods as described herein or as would be applied in a known fashion.

The one or more programs used to implement the systems, methods, and/or interfaces described herein may be provided using any programmable language, e.g., a high-level procedural and/or object orientated programming language that is suitable for communicating with a computer system. Any such programs may, for example, be stored on any suitable device, e.g., a storage media, that is readable by a general or special purpose program running on a computer system (e.g., including processing apparatus) for configuring and operating the computer system when the suitable device is read for performing the procedures described herein. In other words, at least in one embodiment, the illustrative systems, methods, and interfaces may be implemented using a computer readable storage medium, configured with a computer program, where the storage medium so configured causes the computer to operate in a specific and predefined manner to perform functions described herein. Further, in at least one embodiment, the illustrative systems, methods, and interfaces may be described as being implemented by logic (e.g., object code) encoded in one or more non-transitory media that includes code for execution and, when executed by a processor or processing circuitry, is operable to perform operations such as the methods, processes, and/or functionality described herein.

The computing apparatus 140 and the remote computing device 160 may be, for example, any fixed or mobile computer system (e.g., a controller, a microcontroller, a personal computer, minicomputer, tablet computer, etc.). The exact configurations of the computing apparatus 140 and the remote computing device 160 are not limiting, and essentially any device capable of providing suitable computing capabilities and control capabilities (e.g., signal analysis, mathematical functions such as medians, modes, averages, maximum value determination, minimum value determination, slope determination, minimum slope determination, maximum slope determination, graphics processing, etc.) may be used. As described herein, a digital file may be any medium (e.g., volatile or non-volatile memory, a CD-ROM, a punch card, magnetic recordable tape, etc.) containing digital bits (e.g., encoded in binary, trinary, etc.) that may be readable and/or writeable by the computing apparatus 140 and the remote computing device 160 described herein. Also, as described herein, a file in user-readable format may be any representation of data (e.g., ASCII text, binary numbers, hexadecimal numbers, decimal numbers, graphically, etc.) presentable on any medium (e.g., paper, a display, etc.) readable and/or understandable by a user.

In view of the above, it will be readily apparent that the functionality as described in one or more embodiments according to the present disclosure may be implemented in any manner as would be known to one skilled in the art. As such, the computer language, the computer system, or any other software/hardware which is to be used to implement the processes described herein shall not be limiting on the scope of the systems, processes, or programs (e.g., the functionality provided by such systems, processes, or programs) described herein.

The illustrative electrode apparatus 110 may be configured to measure body-surface potentials of a patient 14 and, more particularly, torso-surface potentials of a patient 14. As shown in FIG. 2, the illustrative electrode apparatus 110 may include a set, or array, of external electrodes 112, a strap 113, and interface/amplifier circuitry 116. The electrodes 112 may be attached, or coupled, to the strap 113 and the strap 113 may be configured to be wrapped around the torso of a patient 14 such that the electrodes 112 surround the patient's heart. As further illustrated, the electrodes 112 may be positioned around the circumference of a patient 14, including the posterior, lateral, posterolateral, anterolateral, and anterior locations of the torso of a patient 14.

The illustrative electrode apparatus 110 may be further configured to measure, or monitor, sounds from at least one or both the patient 14. As shown in FIG. 2, the illustrative electrode apparatus 110 may include a set, or array, of acoustic sensors 120 attached, or coupled, to the strap 113. The strap 113 may be configured to be wrapped around the torso of a patient 14 such that the acoustic sensors 120 surround the patient's heart. As further illustrated, the acoustic sensors 120 may be positioned around the circumference of a patient 14, including the posterior, lateral, posterolateral, anterolateral, and anterior locations of the torso of a patient 14.

Further, the electrodes 112 and the acoustic sensors 120 may be electrically connected to interface/amplifier circuitry 116 via wired connection 118. The interface/amplifier circuitry 116 may be configured to amplify the signals from the electrodes 112 and the acoustic sensors 120 and provide the signals to one or both of the computing apparatus 140 and the remote computing device 160. Other illustrative systems may use a wireless connection to transmit the signals sensed by electrodes 112 and the acoustic sensors 120 to the interface/amplifier circuitry 116 and, in turn, to one or both of the computing apparatus 140 and the remote computing device 160, e.g., as channels of data. In one or more embodiments, the interface/amplifier circuitry 116 may be electrically coupled to the computing apparatus 140 using, e.g., analog electrical connections, digital electrical connections, wireless connections, bus-based connections, network-based connections, internet-based connections, etc.

Although in the example of FIG. 2 the electrode apparatus 110 includes a strap 113, in other examples any of a variety of mechanisms, e.g., tape or adhesives, may be employed to aid in the spacing and placement of electrodes 112 and the acoustic sensors 120. In some examples, the strap 113 may include an elastic band, strip of tape, or cloth. Further, in some examples, the strap 113 may be part of, or integrated with, a piece of clothing such as, e.g., a t-shirt. In other examples, the electrodes 112 and the acoustic sensors 120 may be placed individually on the torso of a patient 14. Further, in other examples, one or both of the electrodes 112 (e.g., arranged in an array) and the acoustic sensors 120 (e.g., also arranged in an array) may be part of, or located within, patches, vests, and/or other manners of securing the electrodes 112 and the acoustic sensors 120 to the torso of the patient 14. Still further, in other examples, one or both of the electrodes 112 and the acoustic sensors 120 may be part of, or located within, two sections of material or two patches. One of the two patches may be located on the anterior side of the torso of the patient 14 (to, e.g., monitor electrical signals representative of the anterior side of the patient's heart, measure surrogate cardiac electrical activation times representative of the anterior side of the patient's heart, monitor or measure sounds of the anterior side of the patient, etc.) and the other patch may be located on the posterior side of the torso of the patient 14 (to, e.g., monitor electrical signals representative of the posterior side of the patient's heart, measure surrogate cardiac electrical activation times representative of the posterior side of the patient's heart, monitor or measure sounds of the posterior side of the patient, etc.). And still further, in other examples, one or both of the electrodes 112 and the acoustic sensors 120 may be arranged in a top row and bottom row that extend from the anterior side of the patient 14 across the left side of the patient 14 to the posterior side of the patient 14. Yet still further, in other examples, one or both of the electrodes 112 and the acoustic sensors 120 may be arranged in a curve around the armpit area and may have an electrode/sensor-density that less dense on the right thorax that the other remaining areas.

The electrodes 112 may be configured to surround the heart of the patient 14 and record, or monitor, the electrical signals associated with the depolarization and repolarization of the heart after the signals have propagated through the torso of a patient 14. Each of the electrodes 112 may be used in a unipolar configuration to sense the torso-surface potentials that reflect the cardiac signals. The interface/amplifier circuitry 116 may also be coupled to a return or indifferent electrode (not shown) that may be used in combination with each electrode 112 for unipolar sensing.

In some examples, there may be about 12 to about 50 electrodes 112 and about 12 to about 50 acoustic sensors 120 spatially distributed around the torso of a patient. Other configurations may have more or fewer electrodes 112 and more or fewer acoustic sensors 120. It is to be understood that the electrodes 112 and acoustic sensors 120 may not be arranged or distributed in an array extending all the way around or completely around the patient 14. Instead, the electrodes 112 and acoustic sensors 120 may be arranged in an array that extends only part of the way or partially around the patient 14. For example, the electrodes 112 and acoustic sensors 120 may be distributed on the anterior, posterior, and left sides of the patient with less or no electrodes and acoustic sensors proximate the right side (including posterior and anterior regions of the right side of the patient).

The computing apparatus 140 may record and analyze the torso-surface potential signals sensed by electrodes 112 and the sound signals sensed by the acoustic sensors 120, which are amplified/conditioned by the interface/amplifier circuitry 116. The computing apparatus 140 may be configured to analyze the electrical signals from the electrodes 112 to provide electrocardiogram (ECG) signals, information, or data from the patient's heart as will be further described herein. The computing apparatus 140 may be configured to analyze the electrical signals from the acoustic sensors 120 to provide sound signals, information, or data from the patient's body and/or devices implanted therein (such as a left ventricular assist device).

Additionally, the computing apparatus 140 and the remote computing device 160 may be configured to provide graphical user interfaces 132, 172 depicting various information related to the electrode apparatus 110 and the data gathered, or sensed, using the electrode apparatus 110. For example, the graphical user interfaces 132, 172 may depict ECGs including QRS complexes obtained using the electrode apparatus 110 and sound data including sound waves obtained using the acoustic sensors 120 as well as other information related thereto. Illustrative systems and methods may noninvasively use the electrical information collected using the electrode apparatus 110 and the sound information collected using the acoustic sensors 120 to evaluate a patient's cardiac health and to evaluate and configure cardiac therapy being delivered to the patient.

Further, the electrode apparatus 110 may further include reference electrodes and/or drive electrodes to be, e.g. positioned about the lower torso of the patient 14, that may be further used by the system 100. For example, the electrode apparatus 110 may include three reference electrodes, and the signals from the three reference electrodes may be combined to provide a reference signal. Further, the electrode apparatus 110 may use of three caudal reference electrodes (e.g., instead of standard references used in a Wilson Central Terminal) to get a "true" unipolar signal with less noise from averaging three caudally located reference signals.

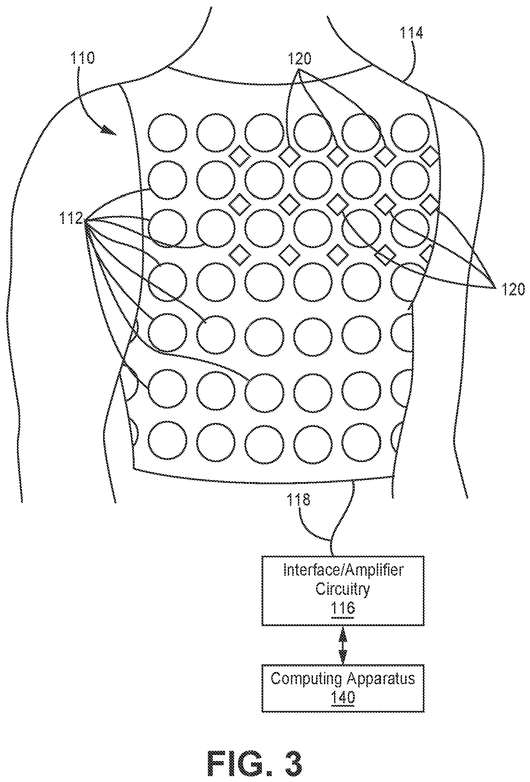

FIG. 3 illustrates another illustrative electrode apparatus 110 that includes a plurality of electrodes 112 configured to surround the heart of the patient 14 and record, or monitor, the electrical signals associated with the depolarization and repolarization of the heart after the signals have propagated through the torso of the patient 14 and a plurality of acoustic sensors 120 configured to surround the heart of the patient 14 and record, or monitor, the sound signals associated with the heart after the signals have propagated through the torso of the patient 14. The electrode apparatus 110 may include a vest 114 upon which the plurality of electrodes 112 and the plurality of acoustic sensors 120 may be attached, or to which the electrodes 112 and the acoustic sensors 120 may be coupled. In at least one embodiment, the plurality, or array, of electrodes 112 may be used to collect electrical information such as, e.g., surrogate electrical activation times. Similar to the electrode apparatus 110 of FIG. 2, the electrode apparatus 110 of FIG. 3 may include interface/amplifier circuitry 116 electrically coupled to each of the electrodes 112 and the acoustic sensors 120 through a wired connection 118 and be configured to transmit signals from the electrodes 112 and the acoustic sensors 120 to computing apparatus 140. As illustrated, the electrodes 112 and the acoustic sensors 120 may be distributed over the torso of a patient 14, including, for example, the posterior, lateral, posterolateral, anterolateral, and anterior locations of the torso of a patient 14.

The vest 114 may be formed of fabric with the electrodes 112 and the acoustic sensors 120 attached to the fabric. The vest 114 may be configured to maintain the position and spacing of electrodes 112 and the acoustic sensors 120 on the torso of the patient 14. Further, the vest 114 may be marked to assist in determining the location of the electrodes 112 and the acoustic sensors 120 on the surface of the torso of the patient 14. In some examples, there may be about 25 to about 256 electrodes 112 and about 25 to about 256 acoustic sensors 120 distributed around the torso of the patient 14, though other configurations may have more or fewer electrodes 112 and more or fewer acoustic sensors 120.

The illustrative systems and methods may be used to provide noninvasive assistance to a user in the evaluation of a patient's cardiac health and/or evaluation and configuration of cardiac therapy being presently delivered to the patient (e.g., by an implantable medical device delivering pacing therapy, by a LVAD, etc.). Further, it is to be understood that the computing apparatus 140 and the remote computing device 160 may be operatively coupled to each other in a plurality of different ways so as to perform, or execute, the functionality described herein. For example, in the embodiment depicted, the computing device 140 may be wireless operably coupled to the remote computing device 160 as depicted by the wireless signal lines emanating therebetween. Additionally, as opposed to wireless connections, one or more of the computing apparatus 140 and the remoting computing device 160 may be operably coupled through one or wired electrical connections.

According to embodiments described herein, the illustrative system 100, which may be referred to as an ECG belt system, may be used with cardiac therapy systems and devices (e.g., CRT pacing devices) to calculate various metrics related to the cardiac health of a patient (e.g., the standard deviation of activation times (SDAT)) across one or more cardiac cycles (or heart beats), and in particular, based on activation times or other data gathered during each QRS event of the cardiac cycle (heart beat). According to various embodiments, the illustrative system 100 may be used to calculate, or generate, electrical heterogeneity information such as, e.g., SDAT, of cardiac cycles during delivery of CRT (e.g., the SDAT for cardiac cycles where CRT paces are delivered). For example, the illustrative system 100 may be used to calculate electrical heterogeneity information for cardiac cycles during biventricular and/or left ventricular pacing. Further, embodiments described herein may be used to evaluate a patient's cardiac health and/or non-CRT pacing. If electrical heterogeneity information is inaccurate, the output of the illustrative system 100 could be misleading, which could potentially impact lead placement (e.g., an implantable lead not being placed at an optimal spot) and/or optimal device programming. For example, if the SDAT is inaccurate, the SDAT may be artificially low, which may cause a clinician to not relocate currently positioned lead as opposed to repositioning the lead to obtain a better response.

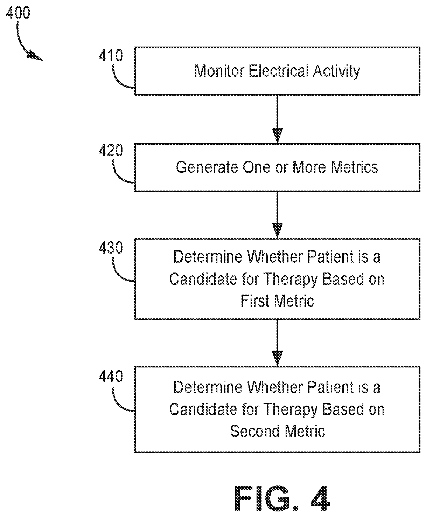

Embodiments described herein may be used to evaluate whether a patient would benefit from a cardiac rhythm therapy (CRT) device. An exemplary method 400 for determining whether a patient is a candidate for a CRT device is shown in FIG. 4 in accordance with embodiments described herein. Electrical activity from tissue of a patient is monitored 410 using a plurality of external electrodes. The plurality of electrodes may be external surface electrodes configured in a band or a vest similar to as described herein with respect to FIGS. 1-3. Each of the electrodes may be positioned or located about the torso of the patient so as to monitor electrical activity (e.g., acquire torso-potentials) from a plurality of different locations about the torso of the patient. Each of the different locations where the electrodes are located may correspond to the electrical activation of different portions or regions of cardiac tissue of the patient's heart. According to various configurations, the plurality of external electrodes comprise a plurality of left external electrodes positioned to the left side of the patient's torso.

One or more cardiac metrics of the patient are generated 420 based on the monitored electrical activity. According to various configurations, the one or more metrics comprise a standard deviation of activation times (SDAT) based on electrical activity recorded from an entire set of plurality of external electrodes and an average of left ventricular activation times (LVAT). In some cases, other measures of dispersion (e.g. standard deviation, range, interquartile range) based on the monitored cardiac electrical activity using the plurality of left external electrodes may be used.

It is determined 430 whether the patient is a candidate for a CRT device based on a first global dyssynchrony metric using the one or more cardiac metrics if the patient has a right bundle branch block.

It is determined 440 whether the patient is a candidate for a CRT device based on a second global dyssynchrony metric using the one or more cardiac metrics if the patient does not have a right bundle branch block.

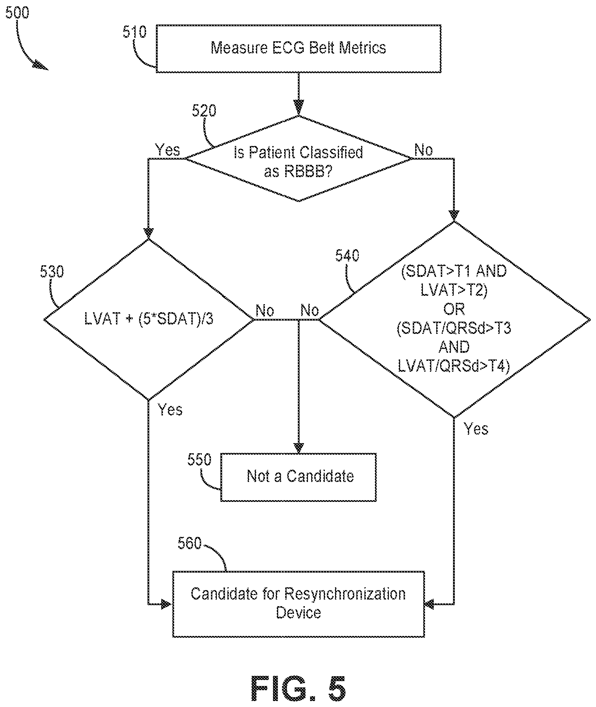

A more detailed method 500 for determining whether a patient is a candidate for a cardiac resynchronization device (CRT) is shown in FIG. 5. Intrinsic ECG belt metrics are measured 510. The metrics may comprise, for example, an SDAT and an LVAT.

It is determined 520 whether the patient is classified as having a right bundle branch block. This may be determined by a doctor in a separate process, for example. In some cases, the system may determine whether the patient has a right bundle branch as a part of the same process as determining whether the patient is a candidate for a CRT device.

If it is determined 520 that the patient is classified as having a right bundle branch block, a first global dyssynchrony metric is determined 530. The first global dyssynchrony metric may be a sum of the LVAT and a fraction of the SDAT. It may be determined that the patient is a candidate for a CRT device if the first global dyssynchrony metric is greater than a predetermined threshold. According to various configurations, the fraction of the SDAT is (5*SDAT)/3 as shown in FIG. 5. The predetermined threshold may be in a range of about 30 ms to about 80 ms. In some cases, the predetermined threshold is about 50 ms.

If it is determined 520 that the patient is not classified as having a right bundle branch block, a second global dyssynchrony metric is determined 540. The second global dyssynchrony metric may comprise determining whether the SDAT is greater than a first predetermined threshold and determining whether the LVAT is greater than a second predetermined threshold. The first predetermined threshold may be in a range of about 15 ms to about 30 ms. In some cases, the first predetermined threshold is about 25 ms. The second predetermined threshold may be in a range of about 25 ms to about 40 ms. In some cases, the second predetermined threshold is about 35 ms. According to various configurations, the second global dyssynchrony metric comprises determining whether the SDAT divided by a QRSd is greater than a third predetermined threshold and determining whether the LVAT divided by the QRSd is greater than a fourth predetermined threshold. The third predetermined threshold may be in a range of about 0.05 to about 0.25. In some cases, the third predetermined threshold is about 0.15. The fourth predetermined threshold may be in a range of about 0.10 ms to about 0.30. In some cases, the fourth predetermined threshold is about 0.20.

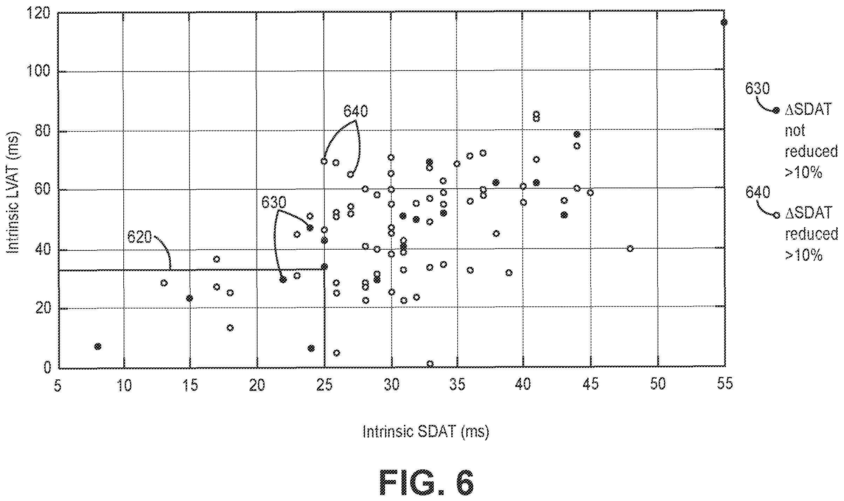

FIG. 6 shows a graph of intrinsic SDAT vs intrinsic LVAT for 115 patients. The data points are color coded light 630 or dark 640 based on the ability to electrically resynchronize or not resynchronize, respectively, during CRT pacing, based on at least a 10% reduction in SDAT. The box 620 indicates patients with intrinsic SDAT<25 ms and intrinsic LVAT<35 ms (a normal level of SDAT/LVAT). About 45% of the patients within this level of intrinsic SDAT/LVAT did not resynchronize electrically whereas a much larger percentage (about 90%) of patients outside the box resynchronized electrically. This implies higher likelihood of resynchronization in patients whose intrinsic SDAT or intrinsic LVAT are greater than normal levels.

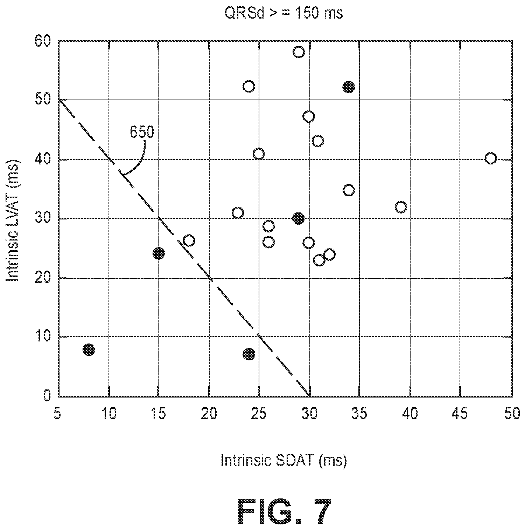

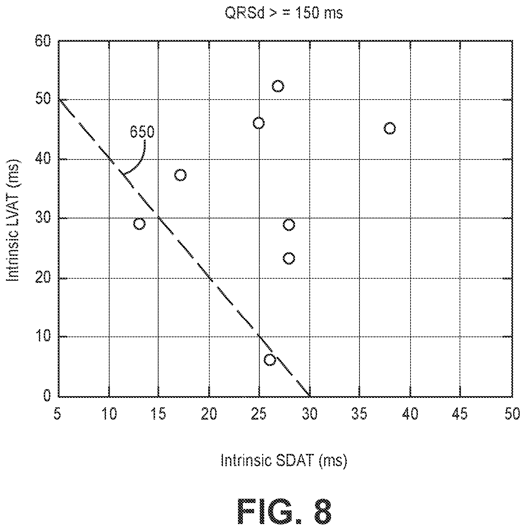

FIGS. 7 and 8 shows the intrinsic SDAT/LVAT distributions for right bundle branch block (RBBB) patients with baseline QRS>150 ms and those with baseline QRS<150 ms, respectively. Patients above the linear dotted line 650 were more likely to resynchonize based on a reduction in SDAT by at least 10% from intrinsic to CRT pacing. A baseline dyssynchrony metric based on a linear combination of intrinsic SDAT and LVAT being above a certain threshold (e.g. LVAT +5/3*SDAT>50 ms) would be a screening metric for RBBB patients that are likely going to resynchronize with CRT. This metric exceeds the threshold even if patients have an intrinsic LVAT that is relatively lower but a higher SDAT and/or if they have a relatively lower SDAT but a higher intrinsic LVAT contributing to enhanced left ventricular dyyssynchrony that leaves room for correction by CRT.

The ECG belt is configured to provide metrics of electrical dyssynchrony. Changes in electrical dyssynchrony from intrinsic to CRT and/or LV pacing have been shown to be useful for titrating LV lead location, optimizing device programming parameters (for example, vectors and/or timing) in bradycardia pacing, for example. The use of ECG belt in a brady population to determine an effective pacing therapy is described. His bundle and/or LBB-area pacing vs traditional RV lead or a combination of both, and/or different locations of RV lead is described herein.

In some cases, ventricle from atrium (VfA) pacing may be used for bradycardia pacing. VfA pacing can be described as providing a synchronized homogeneous activation of ventricles of the heart. As an example, patients with atrial-ventricular (AV) block or prolonged AV timings that can lead to heart failure who have otherwise intact (e.g., normal) QRS can benefit from VfA pacing therapy. In addition, as an example, VfA pacing may provide beneficial activation for heart failure patients with intrinsic ventricular conduction disorders. Further, proper placement of VfA pacing can provide optimal activation of the ventricles for such patients. Further, left ventricular (LV) resynchronization for heart failure patients with left bundle branch block (LBBB) may find that VfA pacing enables easier access to left ventricular endocardium without exposing the leadless device or lead to endocardial blood pool. At the same time, in that example, this can help engage part of the conduction system to potentially correct LBBB and effectively resynchronize the patient.

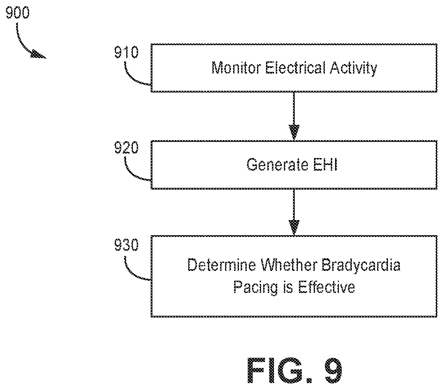

An exemplary method 900 for determining whether bradycardia pacing therapy is effective is shown in FIG.9. Electrical activity from tissue of a patient is monitored 910 using a plurality of external electrodes. According to various configurations, the plurality of external electrodes comprise a plurality of left external electrodes positioned to the left side of the patient's torso.

Electrical heterogeneity information (EHI) is generated 920 based on the monitored cardiac electrical activity during delivery of bradycardia pacing to the patient's heart. The EHI may be generated based on one or more metrics comprising an SDAT, an LVAT, a LV disp metric based on standard deviation of activation times of electrodes on the left side of the body reflecting left ventricular activation, and an RV disp metric based on standard deviation of activation times of electrodes on the right side of the body reflecting right ventricular activation time. According to various configurations, the metrics may include QRS duration, peak of timing on lead V6, and/or changes in morphology on precordial leads V1, V2, for example.

It is determined 930 whether the bradycardia pacing therapy is effective based on the generated EHI. According to various configurations, determining whether the bradycardia pacing therapy is effective comprises determining if the bradycardia pacing is reflective of normalization of conduction for a population of patients during bradycardia pacing. In some cases, the population of patients has at least one similar characteristic to the patient. For example, the at least one characteristic may include age, sex, height, and weight.

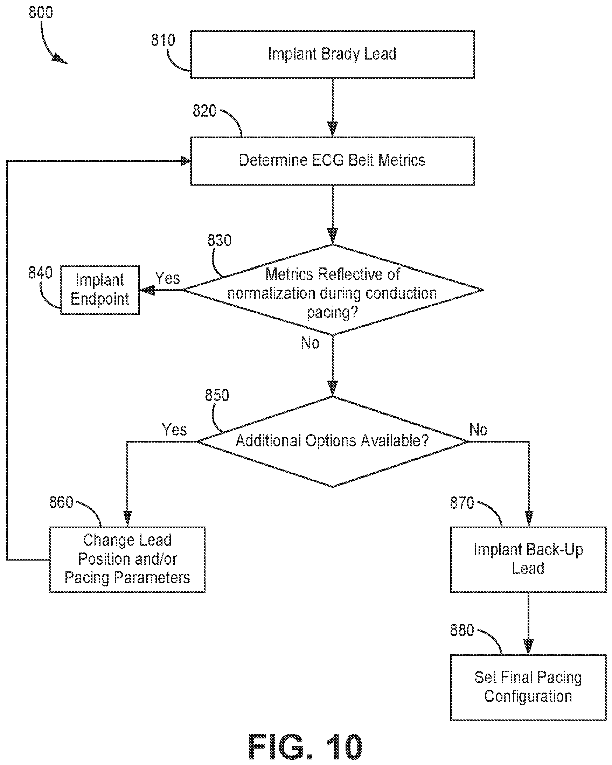

FIG. 10 shows another method for determining whether bradycardia pacing therapy is effective. A brady lead is implanted 810. For example, His area, LBB area, ventricle from atrium, and/or septal vein leads.

One or more ECG belt metrics are determined 820. The ECG belt metrics may include one or both of an SDAT, LVAT and an EHI. It is determined 830 if one or more of the determined metrics are reflective of normalization during conduction pacing. If it is determined 830 that the metrics are reflective of normalization, the process ends 840

If it is determined 830 that the metrics are not reflective of normalization during conduction pacing, it is determined 850 if different options are available. One option may include changing lead position. The different options may include one or more of changing lead position and/or changing pacing parameters. The pacing parameters may comprise one or more of A-V delay, pulse width, amplitude, voltage, and/or burst length.

A position of an RV lead and/or a pace timing may be changed to minimize dyssynchrony during RV pacing. For example, a position of the RV lead can be moved between RVOT, RV apex, and RV mid-septal.

In cases in which His area, LBB area pacing are used, the lead location and/or the AV/VV delay may me changed to minimize dyssynchrony. A back-up RV lead may be used in His bundle pacing, for example, and the location of the back-up RV lead may also be optimized to minimize dyssynchrony.

Lead position and/or timing for dual bundle pacing may also be positioned to minimize dyssynchrony. The two bundle leads may be positioned in the His bundle proximal and the other more distal in the left bundle. A single multipolar lead may be used for dual bundle pacing. For example, in left bundle branch block (LBBB) patients, such as patients that have a prolonged PR where intrinsic right bundle fusion is not possible, a single multipolar lead may be used.

The lead location and/or timing of pacing may be used for a lead located in the septal vein. This may be used to provide feedback on engagement of the conduction system from pacing from locations within the septal performator branch of the great cardiac vein.

Embodiments described herein may be used to optimize timing of biventricular intraseptal pacing. This may include both the RV and the LV septum.

If it is determined that different options are available, lead position and/or pacing parameters are changed. After, the lead position and/or the pacing parameters are changed, the process continues with measuring 820 the ECG belt metrics.

If it is determined that different options are not available, one or more back-up leads are implanted 870. The final pacing configuration and parameters are set 880 based on the lowest SDAT. Absolute values of SDAT may be determined during various pacing configuration, timing and other parameters (e.g. pacing from both leads with different AV timing, VV timing, outputs, etc) and the set of parameters and pacing configuration that yields the lowest absolute value of SDAT may be selected for final programming.

Various embodiments may include pacing at different locations. For example, the locations may include a conduction system and/or a septum. A conduction system pacing lead and/or a traditional pacing lead (e.g. RV lead) with different AV/VV timings and other pacing parameters. The pacing locations and/or parameters may be selected based on which produces the lowest electrical dyssynchrony. In some cases, pacing locations and/or parameters that produce metrics that are representative of normal levels of values of that particular metric, indicating 'normalization' by paced activation, may be selected.

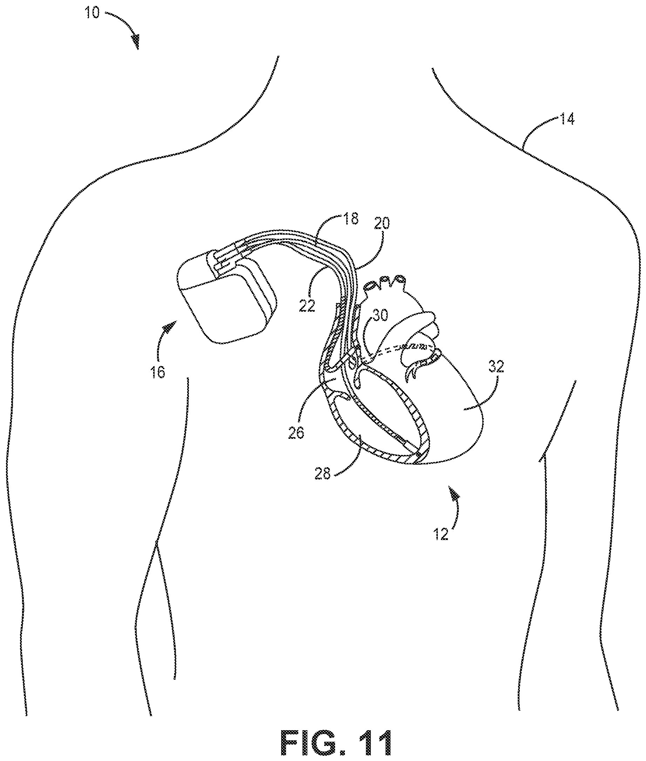

Illustrative cardiac therapy systems and devices may be further described herein with reference to FIGS. 11-13 that may utilizes the illustrative systems, interfaces, methods, and processes described herein with respect to FIGS. 1-10.

FIG. 11 is a conceptual diagram illustrating an illustrative therapy system 10 that may be used to deliver pacing therapy to a patient 14. Patient 14 may, but not necessarily, be a human. The therapy system 10 may include an implantable medical device 16 (IMD), which may be coupled to leads 18, 20, 22. The IMD 16 may be, e.g., an implantable pacemaker, cardioverter, and/or defibrillator, that delivers, or provides, electrical signals (e.g., paces, etc.) to and/or senses electrical signals from the heart 12 of the patient 14 via electrodes coupled to one or more of the leads 18, 20, 22.

The leads 18, 20, 22 extend into the heart 12 of the patient 14 to sense electrical activity of the heart 12 and/or to deliver electrical stimulation to the heart 12. In the example shown in FIG. 11, the right ventricular (RV) lead 18 extends through one or more veins (not shown), the superior vena cava (not shown), and the right atrium 26, and into the right ventricle 28. The left ventricular (LV) coronary sinus lead 20 extends through one or more veins, the vena cava, the right atrium 26, and into the coronary sinus 30 to a region adjacent to the free wall of the left ventricle 32 of the heart 12. The right atrial (RA) lead 22 extends through one or more veins and the vena cava, and into the right atrium 26 of the heart 12.

The IMD 16 may sense, among other things, electrical signals attendant to the depolarization and repolarization of the heart 12 via electrodes coupled to at least one of the leads 18, 20, 22. In some examples, the IMD 16 provides pacing therapy (e.g., pacing pulses) to the heart 12 based on the electrical signals sensed within the heart 12. The IMD 16 may be operable to adjust one or more parameters associated with the pacing therapy such as, e.g., A-V delay and other various timings, pulse wide, amplitude, voltage, burst length, etc. Further, the IMD 16 may be operable to use various electrode configurations to deliver pacing therapy, which may be unipolar, bipolar, quadripolar, or further multipolar. For example, a multipolar lead may include several electrodes that can be used for delivering pacing therapy. Hence, a multipolar lead system may provide, or offer, multiple electrical vectors to pace from. A pacing vector may include at least one cathode, which may be at least one electrode located on at least one lead, and at least one anode, which may be at least one electrode located on at least one lead (e.g., the same lead, or a different lead) and/or on the casing, or can, of the IMD. While improvement in cardiac function as a result of the pacing therapy may primarily depend on the cathode, the electrical parameters like impedance, pacing threshold voltage, current drain, longevity, etc. may be more dependent on the pacing vector, which includes both the cathode and the anode. The IMD 16 may also provide defibrillation therapy and/or cardioversion therapy via electrodes located on at least one of the leads 18, 20, 22. Further, the IMD 16 may detect arrhythmia of the heart 12, such as fibrillation of the ventricles 28, 32, and deliver defibrillation therapy to the heart 12 in the form of electrical pulses. In some examples, IMD 16 may be programmed to deliver a progression of therapies, e.g., pulses with increasing energy levels, until a fibrillation of heart 12 is stopped.

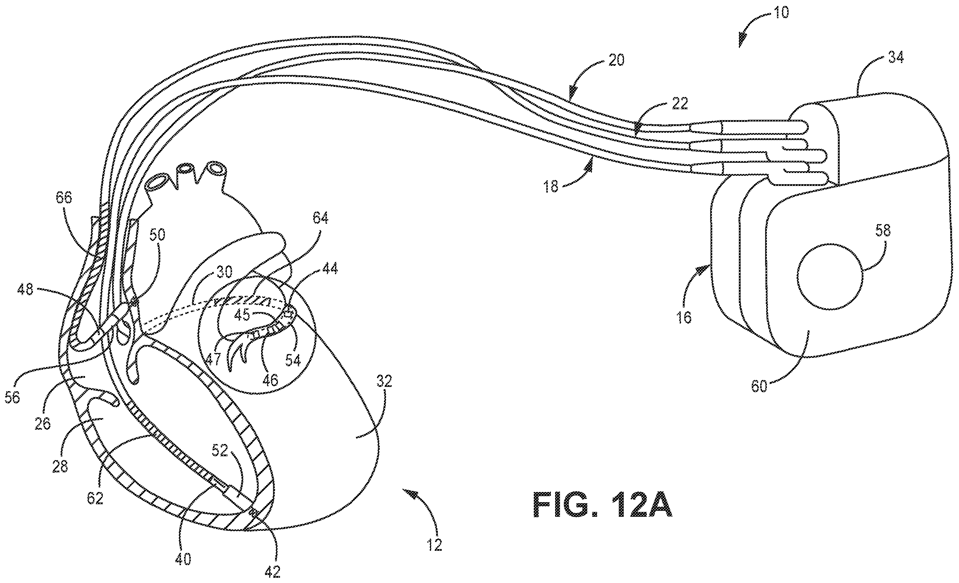

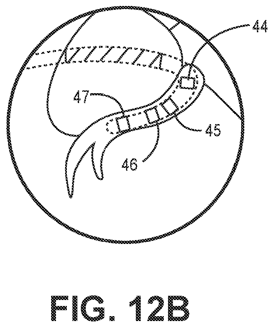

FIGS. 12A-12B are conceptual diagrams illustrating the IMD 16 and the leads 18, 20, 22 of therapy system 10 of FIG. 11 in more detail. The leads 18, 20, 22 may be electrically coupled to a therapy delivery module (e.g., for delivery of pacing therapy), a sensing module (e.g., for sensing one or more signals from one or more electrodes), and/or any other modules of the IMD 16 via a connector block 34. In some examples, the proximal ends of the leads 18, 20, 22 may include electrical contacts that electrically couple to respective electrical contacts within the connector block 34 of the IMD 16. In addition, in some examples, the leads 18, 20, 22 may be mechanically coupled to the connector block 34 with the aid of set screws, connection pins, or another suitable mechanical coupling mechanism.

Each of the leads 18, 20, 22 includes an elongated insulative lead body, which may carry a number of conductors (e.g., concentric coiled conductors, straight conductors, etc.) separated from one another by insulation (e.g., tubular insulative sheaths). In the illustrated example, bipolar electrodes 40, 42 are located proximate to a distal end of the lead 18. In addition, bipolar electrodes 44, 45, 46, 47 are located proximate to a distal end of the lead 20 and bipolar electrodes 48, 50 are located proximate to a distal end of the lead 22.

The electrodes 40, 44, 45, 46, 47, 48 may take the form of ring electrodes, and the electrodes 42, 50 may take the form of extendable helix tip electrodes mounted retractably within the insulative electrode heads 52, 54, 56, respectively. Each of the electrodes 40, 42, 44, 45, 46, 47, 48, 50 may be electrically coupled to a respective one of the conductors (e.g., coiled and/or straight) within the lead body of its associated lead 18, 20, 22, and thereby coupled to a respective one of the electrical contacts on the proximal end of the leads 18, 20, 22.

Additionally, electrodes 44, 45, 46 and 47 may have an electrode surface area of about 5.3 mm² to about 5.8 mm². Electrodes 44, 45, 46, and 47 may also be referred to as LV1, LV2, LV3, and LV4, respectively. The LV electrodes (i.e., left ventricle electrode 1 (LV1) 44, left ventricle electrode 2 (LV2) 45, left ventricle electrode 3 (LV3) 46, and left ventricle 4 (LV4) 47 etc.) on the lead 20 can be spaced apart at variable distances. For example, electrode 44 may be a distance of, e.g., about 21 millimeters (mm), away from electrode 45, electrodes 45 and 46 may be spaced a distance of, e.g. about 1.3 mm to about 1.5 mm, away from each other, and electrodes 46 and 47 may be spaced a distance of, e.g. 20 mm to about 21 mm, away from each other.

The electrodes 40, 42, 44, 45, 46, 47, 48, 50 may further be used to sense electrical signals (e.g., morphological waveforms within electrograms (EGM)) attendant to the depolarization and repolarization of the heart 12. The electrical signals are conducted to the IMD 16 via the respective leads 18, 20, 22. In some examples, the IMD 16 may also deliver pacing pulses via the electrodes 40, 42, 44, 45, 46, 47, 48, 50 to cause depolarization of cardiac tissue of the patient's heart 12. In some examples, as illustrated in FIG. 12A, the IMD 16 includes one or more housing electrodes, such as housing electrode 58, which may be formed integrally with an outer surface of a housing 60 (e.g., hermetically-sealed housing) of the IMD 16 or otherwise coupled to the housing 60. Any of the electrodes 40, 42, 44, 45, 46, 47, 48, 50 may be used for unipolar sensing or pacing in combination with the housing electrode 58. It is generally understood by those skilled in the art that other electrodes can also be selected to define, or be used for, pacing and sensing vectors. Further, any of electrodes 40, 42, 44, 45, 46, 47, 48, 50, 58, when not being used to deliver pacing therapy, may be used to sense electrical activity during pacing therapy.

As described in further detail with reference to FIG. 12A, the housing 60 may enclose a therapy delivery module that may include a stimulation generator for generating cardiac pacing pulses and defibrillation or cardioversion shocks, as well as a sensing module for monitoring the electrical signals of the patient's heart (e.g., the patient's heart rhythm). The leads 18, 20, 22 may also include elongated electrodes 62, 64, 66, respectively, which may take the form of a coil. The IMD 16 may deliver defibrillation shocks to the heart 12 via any combination of the elongated electrodes 62, 64, 66 and the housing electrode 58. The electrodes 58, 62, 64, 66 may also be used to deliver cardioversion pulses to the heart 12. Further, the electrodes 62, 64, 66 may be fabricated from any suitable electrically conductive material, such as, but not limited to, platinum, platinum alloy, and/or other materials known to be usable in implantable defibrillation electrodes. Since electrodes 62, 64, 66 are not generally configured to deliver pacing therapy, any of electrodes 62, 64, 66 may be used to sense electrical activity and may be used in combination with any of electrodes 40, 42, 44, 45, 46, 47, 48, 50, 58. In at least one embodiment, the RV elongated electrode 62 may be used to sense electrical activity of a patient's heart during the delivery of pacing therapy (e.g., in combination with the housing electrode 58, or defibrillation electrode-to-housing electrode vector).

The configuration of the illustrative therapy system 10 illustrated in FIGS. 11-13 is merely one example. In other examples, the therapy system may include epicardial leads and/or patch electrodes instead of or in addition to the transvenous leads 18, 20, 22 illustrated in FIG. 11. Additionally, in other examples, the therapy system 10 may be implanted in/around the cardiac space without transvenous leads (e.g., leadless/wireless pacing systems) or with leads implanted (e.g., implanted transvenously or using approaches) into the left chambers of the heart (in addition to or replacing the transvenous leads placed into the right chambers of the heart as illustrated in FIG. 11). Further, in one or more embodiments, the IMD 16 need not be implanted within the patient 14. For example, the IMD 16 may deliver various cardiac therapies to the heart 12 via percutaneous leads that extend through the skin of the patient 14 to a variety of positions within or outside of the heart 12. In one or more embodiments, the system 10 may utilize wireless pacing (e.g., using energy transmission to the intracardiac pacing component(s) via ultrasound, inductive coupling, RF, etc.) and sensing cardiac activation using electrodes on the can/housing and/or on subcutaneous leads.

In other examples of therapy systems that provide electrical stimulation therapy to the heart 12, such therapy systems may include any suitable number of leads coupled to the IMD 16, and each of the leads may extend to any location within or proximate to the heart 12. For example, other examples of therapy systems may include three transvenous leads located as illustrated in FIGS. 11-13. Still further, other therapy systems may include a single lead that extends from the IMD 16 into the right atrium 26 or the right ventricle 28, or two leads that extend into a respective one of the right atrium 26 and the right ventricle 28.

According to various configurations, VfA pacing may be used. Further illustrative systems, methods, and processes for optimizing the cardiac pacing therapy may be described in U.S. Pat. App. Ser. No. 15/934,517 filed on March 23, 2019 entitled "Evaluation of Ventricle from Atrium Pacing Therapy" and U.S. Prov. Pat. App. Ser. No. 62/725,763 filed on August 31, 2018 entitled "Adaptive VFA Cardiac Therapy,".

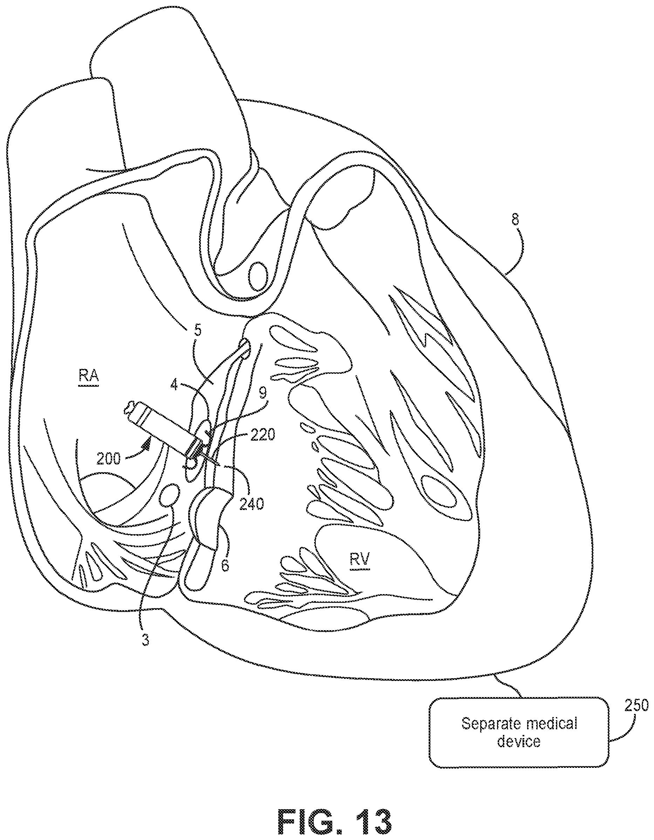

An illustrative VfA cardiac therapy system is depicted in FIG. 13 that may be configured to be used with, for example, the systems and methods described herein with respect to FIGS. 1-10. Although it is to be understood that the present disclosure may utilize one or both of leadless and leaded implantable medical devices, the illustrative cardiac therapy system of FIG. 13 includes a leadless intracardiac medical device 10 that may be configured for single or dual chamber therapy and implanted in a patient's heart 8. In some embodiments, the device 200 may be configured for single chamber pacing and may, for example, switch between single chamber and multiple chamber pacing (e.g., dual or triple chamber pacing). As used herein, "intracardiac" refers to a device configured to be implanted entirely within a patient's heart, for example, to provide cardiac therapy. The device 200 is shown implanted in the right atrium (RA) of the patient's heart 8 in a target implant region 4. The device 200 may include one or more fixation members 20 that anchor a distal end of the device 200 against the atrial endocardium in a target implant region 4. The target implant region 4 may lie between the Bundle of His 5 and the coronary sinus 3 and may be adjacent, or next to, the tricuspid valve 6. The device 200 may be described as a ventricle-from-atrium device because, for example, the device 200 may perform, or execute, one or both of sensing electrical activity from and providing therapy to one or both ventricles (e.g., right ventricle, left ventricle, or both ventricles, depending on the circumstances) while being generally disposed in the right atrium. In particular, the device 200 may include a tissue-piercing electrode that may be implanted in the basal and/or septal region of the left ventricular myocardium of the patient's heart from the triangle of Koch region of the right atrium through the right atrial endocardium and central fibrous body.

The device 200 may be described as a leadless implantable medical device. As used herein, "leadless" refers to a device being free of a lead extending out of the patient's heart 8. Further, although a leadless device may have a lead, the lead would not extend from outside of the patient's heart to inside of the patient's heart or would not extend from inside of the patient's heart to outside of the patient's heart. Some leadless devices may be introduced through a vein, but once implanted, the device is free of, or may not include, any transvenous lead and may be configured to provide cardiac therapy without using any transvenous lead. Further, a leadless VfA device, in particular, does not use a lead to operably connect to an electrode in the ventricle when a housing of the device is positioned in the atrium. Additionally, a leadless electrode may be coupled to the housing of the medical device without using a lead between the electrode and the housing.

The device 200 may include a dart electrode assembly 12 defining, or having, a straight shaft extending from a distal end region of device 200. The dart electrode assembly 220 may be placed, or at least configured to be placed, through the atrial myocardium and the central fibrous body and into the ventricular myocardium 240, or along the ventricular septum, without perforating entirely through the ventricular endocardial or epicardial surfaces. The dart electrode assembly 220 may carry, or include, an electrode at a distal end region of the shaft such that the electrode may be positioned within the ventricular myocardium for sensing ventricular signals and delivering ventricular pacing pulses (e.g., to depolarize the left ventricle and/or right ventricle to initiate a contraction of the left ventricle and/or right ventricle). In some examples, the electrode at the distal end region of the shaft is a cathode electrode provided for use in a bipolar electrode pair for pacing and sensing. While the implant region 4 as illustrated may enable one or more electrodes of the dart electrode assembly 220 to be positioned in the ventricular myocardium, it is recognized that a device having the aspects disclosed herein may be implanted at other locations for multiple chamber pacing (e.g., dual or triple chamber pacing), single chamber pacing with multiple chamber sensing, single chamber pacing and/or sensing, or other clinical therapy and applications as appropriate.

It is to be understood that although device 200 is described herein as including a single dart electrode assembly, the device 200 may include more than one dart electrode assembly placed, or configured to be placed, through the atrial myocardium and the central fibrous body, and into the ventricular myocardium 240, or along the ventricular septum, without perforating entirely through the ventricular endocardial or epicardial surfaces. Additionally, each dart electrode assembly may carry, or include, more than a single electrode at the distal end region, or along other regions (e.g., proximal or central regions), of the shaft.

The cardiac therapy system may also include a separate medical device 250 (depicted diagrammatically in FIG. 13), which may be positioned outside the patient's heart 8 (e.g., subcutaneously) and may be operably coupled to the patient's heart 8 to deliver cardiac therapy thereto. In one example, separate medical device 250 may be an extravascular ICD. In some embodiments, an extravascular ICD may include a defibrillation lead including, or carrying, a defibrillation electrode. A therapy vector may exist between the defibrillation electrode on the defibrillation lead and a housing electrode of the ICD. Further, one or more electrodes of the ICD may also be used for sensing electrical signals related to the patient's heart 8. The ICD may be configured to deliver shock therapy including one or more defibrillation or cardioversion shocks. For example, if an arrhythmia is sensed, the ICD may send a pulse via the electrical lead wires to shock the heart and restore its normal rhythm. In some examples, the ICD may deliver shock therapy without placing electrical lead wires within the heart or attaching electrical wires directly to the heart (subcutaneous ICDs). Examples of extravascular, subcutaneous ICDs that may be used with the system 2 described herein may be described in U.S. Patent No. 9,278,229 (Reinke et al.), issued 8 March 2016.

In the case of shock therapy (e.g., defibrillation shocks provided by the defibrillation electrode of the defibrillation lead), the separate medical device 50 (e.g., extravascular ICD) may include a control circuit that uses a therapy delivery circuit to generate defibrillation shocks having any of a number of waveform properties, including leading-edge voltage, tilt, delivered energy, pulse phases, and the like. The therapy delivery circuit may, for instance, generate monophasic, biphasic, or multiphasic waveforms. Additionally, the therapy delivery circuit may generate defibrillation waveforms having different amounts of energy. For example, the therapy delivery circuit may generate defibrillation waveforms that deliver a total of between approximately 60-80 Joules (J) of energy for subcutaneous defibrillation.

The separate medical device 250 may further include a sensing circuit. The sensing circuit may be configured to obtain electrical signals sensed via one or more combinations of electrodes and to process the obtained signals. The components of the sensing circuit may include analog components, digital components, or a combination thereof. The sensing circuit may, for example, include one or more sense amplifiers, filters, rectifiers, threshold detectors, analog-to-digital converters (ADCs), or the like. The sensing circuit may convert the sensed signals to digital form and provide the digital signals to the control circuit for processing and/or analysis. For example, the sensing circuit may amplify signals from sensing electrodes and convert the amplified signals to multi-bit digital signals by an ADC, and then provide the digital signals to the control circuit. In one or more embodiments, the sensing circuit may also compare processed signals to a threshold to detect the existence of atrial or ventricular depolarizations (e.g., P- or R-waves) and indicate the existence of the atrial depolarization (e.g., P-waves) or ventricular depolarizations (e.g., R-waves) to the control circuit.

The device 200 and the separate medical device 250 may cooperate to provide cardiac therapy to the patient's heart 8. For example, the device 200 and the separate medical device 250 may be used to detect tachycardia, monitor tachycardia, and/or provide tachycardia-related therapy. For example, the device 200 may communicate with the separate medical device 250 wirelessly to trigger shock therapy using the separate medical device 250. As used herein, "wirelessly" refers to an operative coupling or connection without using a metal conductor between the device 200 and the separate medical device 250. In one example, wireless communication may use a distinctive, signaling, or triggering electrical-pulse provided by the device 200 that conducts through the patient's tissue and is detectable by the separate medical device 250. In another example, wireless communication may use a communication interface (e.g., an antenna) of the device 200 to provide electromagnetic radiation that propagates through patient's tissue and is detectable, for example, using a communication interface (e.g., an antenna) of the separate medical device 250.

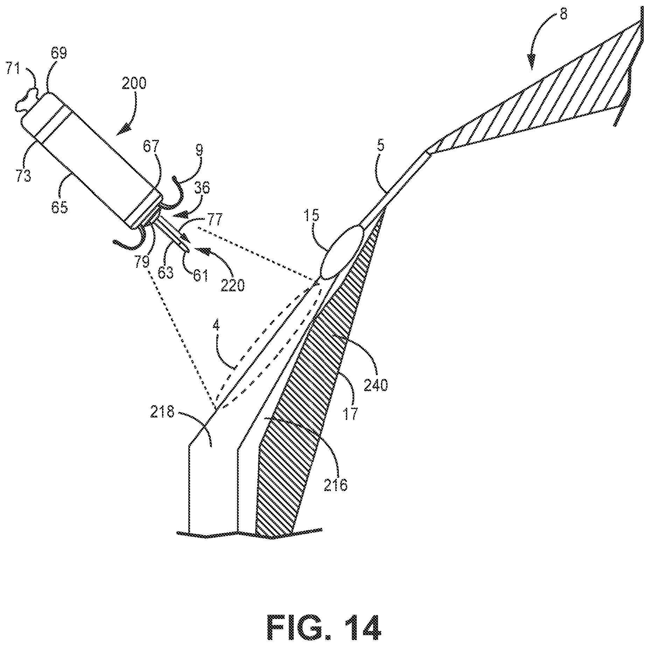

FIG. 14 is an enlarged conceptual diagram of the intracardiac medical device 200 of FIG. 13 and anatomical structures of the patient's heart 8. In particular, the device 200 is configured to sense cardiac signals and/or deliver pacing therapy. The intracardiac device 200 may include a housing 30. The housing 30 may define a hermetically-sealed internal cavity in which internal components of the device 10 reside, such as a sensing circuit, therapy delivery circuit, control circuit, memory, telemetry circuit, other optional sensors, and a power source as generally described in conjunction with FIG. 10. The housing 65 may include (e.g., be formed of or from) an electrically conductive material such as, e.g., titanium or titanium alloy, stainless steel, MP35N (a non-magnetic nickel-cobalt-chromium-molybdenum alloy), platinum alloy, or other bio-compatible metal or metal alloy. In other examples, the housing 65 may include (e.g., be formed of or from) a non-conductive material including ceramic, glass, sapphire, silicone, polyurethane, epoxy, acetyl copolymer plastics, polyether ether ketone (PEEK), a liquid crystal polymer, or other biocompatible polymer.

In at least one embodiment, the housing 65 may be described as extending between a distal end region 67 and a proximal end region 69 and as defining a generally-cylindrical shape, e.g., to facilitate catheter delivery. In other embodiments, the housing 65 may be prismatic or any other shape to perform the functionality and utility described herein. The housing 65 may include a delivery tool interface member 71, e.g., defined, or positioned, at the proximal end region 69, for engaging with a delivery tool during implantation of the device 200.

All or a portion of the housing 65 may function as a sensing and/or pacing electrode during cardiac therapy. In the example shown, the housing 65 includes a proximal housing-based electrode 73 that circumscribes a proximal portion (e.g., closer to the proximal end region 69 than the distal end region 67) of the housing 65. When the housing 65 is (e.g., defines, formed from, etc.) an electrically-conductive material, such as a titanium alloy or other examples listed above, portions of the housing 65 may be electrically insulated by a non-conductive material, such as a coating of parylene, polyurethane, silicone, epoxy, or other biocompatible polymer, leaving one or more discrete areas of conductive material exposed to form, or define, the proximal housing-based electrode 73. When the housing 65 is (e.g., defines, formed from, etc.) a non-conductive material, such as a ceramic, glass or polymer material, an electrically-conductive coating or layer, such as a titanium, platinum, stainless steel, or alloys thereof, may be applied to one or more discrete areas of the housing 65 to form, or define, the proximal housing-based electrode 73. In other examples, the proximal housing-based electrode 73 may be a component, such as a ring electrode, that is mounted or assembled onto the housing 65. The proximal housing-based electrode 73 may be electrically coupled to internal circuitry of the device 200, e.g., via the electrically-conductive housing 65 or an electrical conductor when the housing 65 is a non-conductive material.

In the example shown, the proximal housing-based electrode 73 is located nearer to the housing proximal end region 69 than the housing distal end region 67, and therefore, may be referred to as a proximal housing-based electrode 73. In other examples, however, the proximal housing-based electrode 73 may be located at other positions along the housing 65, e.g., more distal relative to the position shown.

At the distal end region 67, the device 200 may include a distal fixation and electrode assembly 36, which may include one or more fixation members 20 and one or more dart electrode assemblies 12 of equal or unequal length. In one such example as shown, a single dart electrode assembly 12 includes a shaft 63 extending distally away from the housing distal end region 67 and one or more electrode elements, such as a tip electrode 75 at or near the free, distal end region of the shaft 40. The tip electrode 75 may have a conical or hemi-spherical distal tip with a relatively narrow tip-diameter (e.g., less than about 1 millimeter (mm)) for penetrating into and through tissue layers without using a sharpened tip or needle-like tip having sharpened or beveled edges.

The dart electrode assembly 220 may be configured to pierce through one or more tissue layers to position the tip electrode 75 within a desired tissue layer such as, e.g., the ventricular myocardium. As such, the height 77, or length, of the shaft 63 may correspond to the expected pacing site depth, and the shaft 63 may have a relatively-high compressive strength along its longitudinal axis to resist bending in a lateral or radial direction when pressed against and into the implant region 4. If a second dart electrode assembly 220 is employed, its length may be unequal to the expected pacing site depth and may be configured to act as an indifferent electrode for delivering of pacing energy to and/or sensing signals from the tissue. In one embodiment, a longitudinal axial force may be applied against the tip electrode 75, e.g., by applying longitudinal pushing force to the proximal end 69 of the housing 65, to advance the dart electrode assembly 220 into the tissue within the target implant region.

The shaft 63 may be described as longitudinally non-compressive and/or elastically deformable in lateral or radial directions when subjected to lateral or radial forces to allow temporary flexing, e.g., with tissue motion, but may return to its normally straight position when lateral forces diminish. Thus, the dart electrode assembly 220 including the shaft 63 may be described as being resilient. When the shaft 63 is not exposed to any external force, or to only a force along its longitudinal central axis, the shaft 63 may retain a straight, linear position as shown.

In other words, the shaft 63 of the dart electrode assembly 220 may be a normally straight member and may be rigid. In other embodiments, the shaft 63 may be described as being relatively stiff but still possessing limited flexibility in lateral directions. Further, the shaft 63 may be non-rigid to allow some lateral flexing with heart motion. However, in a relaxed state, when not subjected to any external forces, the shaft 63 may maintain a straight position as shown to hold the tip electrode 75 spaced apart from the housing distal end region 67 at least by a height, or length, 77 of the shaft 63.

The one or more fixation members 9 may be described as one or more "tines" having a normally curved position. The tines may be held in a distally extended position within a delivery tool. The distal tips of tines may penetrate the heart tissue to a limited depth before elastically, or resiliently, curving back proximally into the normally curved position (shown) upon release from the delivery tool. Further, the fixation members 20 may include one or more aspects described in, for example, U.S. Patent No. 9,675,579 (Grubac et al.), issued 13 June 2017, and U.S. Patent No. 9,119,959 (Rys et al.), issued 1 September 2015.

In some examples, the distal fixation and electrode assembly 36 includes a distal housing-based electrode 79. In the case of using the device 10 as a pacemaker for multiple chamber pacing (e.g., dual or triple chamber pacing) and sensing, the tip electrode 75 may be used as a cathode electrode paired with the proximal housing-based electrode 73 serving as a return anode electrode. Alternatively, the distal housing-based electrode 79 may serve as a return anode electrode paired with tip electrode 75 for sensing ventricular signals and delivering ventricular pacing pulses. In other examples, the distal housing-based electrode 79 may be a cathode electrode for sensing atrial signals and delivering pacing pulses to the atrial myocardium in the target implant region 4. When the distal housing-based electrode 79 serves as an atrial cathode electrode, the proximal housing-based electrode 73 may serve as the return anode paired with the tip electrode 75 for ventricular pacing and sensing and as the return anode paired with the distal housing-based electrode 79 for atrial pacing and sensing.

As shown in this illustration, the target implant region 4 in some pacing applications is along the atrial endocardium 218, generally inferior to the AV node 15 and the His bundle 5. The dart electrode assembly 220 may at least partially define the height 77, or length, of the shaft 63 for penetrating through the atrial endocardium 218 in the target implant region 4, through the central fibrous body 216, and into the ventricular myocardium 240 without perforating through the ventricular endocardial surface 17. When the height 77, or length, of the dart electrode assembly 220 is fully advanced into the target implant region 4, the tip electrode 75 may rest within the ventricular myocardium 240, and the distal housing-based electrode 79 may be positioned in intimate contact with or close proximity to the atrial endocardium 218. The dart electrode assembly 220 may have a total combined height 77, or length, of tip electrode 75 and shaft 63 from about 3 mm to about 8 mm in various examples. The diameter of the shaft 63 may be less than about 2 mm, and may be about 1 mm or less, or even about 0.6 mm or less.

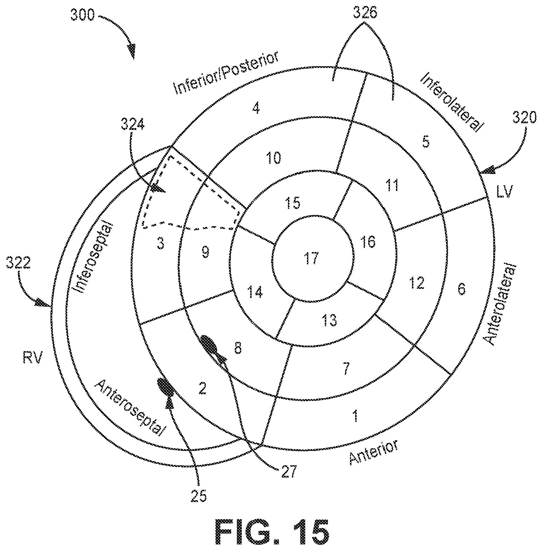

FIG. 15 is a two-dimensional (2D) ventricular map 300 of a patient's heart (e.g., a top-down view) showing the left ventricle 320 in a standard 17 segment view and the right ventricle 322. The map 300 defines, or includes, a plurality of areas 326 corresponding to different regions of a human heart. As illustrated, the areas 326 are numerically labeled 1-17 (which, e.g., correspond to a standard 17 segment model of a human heart, correspond to 17 segments of the left ventricle of a human heart, etc.). Areas 326 of the map 300 may include basal anterior area 1, basal anteroseptal area 2, basal inferoseptal area 3, basal inferior area 4, basal inferolateral area 5, basal anterolateral area 6, mid-anterior area 7, mid-anteroseptal area 8, mid-inferoseptal area 9, mid-inferior area 10, mid-inferolateral area 11, mid-anterolateral area 12, apical anterior area 13, apical septal area 14, apical inferior area 15, apical lateral area 216, and apex area 17. The inferoseptal and anteroseptal areas of the right ventricle 322 are also illustrated, as well as the right bunch branch (RBB) 25 and left bundle branch (LBB) 27.

In some embodiments, any of the tissue-piercing electrodes of the present disclosure may be implanted in the basal and/or septal region of the left ventricular myocardium of the patient's heart. In particular, the tissue-piercing electrode may be implanted from the triangle of Koch region of the right atrium through the right atrial endocardium and central fibrous body. Once implanted, the tissue-piercing electrode may be positioned in the target implant region 4 (FIGS. 7-8), such as the basal and/or septal region of the left ventricular myocardium. With reference to map 300, the basal region includes one or more of the basal anterior area 1, basal anteroseptal area 2, basal inferoseptal area 3, basal inferior area 4, mid-anterior area 7, mid-anteroseptal area 8, mid-inferoseptal area 9, and mid-inferior area 10. With reference to map 300, the septal region includes one or more of the basal anteroseptal area 2, basal anteroseptal area 3, mid-anteroseptal area 8, mid-inferoseptal area 9, and apical septal area 14.

In some embodiments, the tissue-piercing electrode may be positioned in the basal septal region of the left ventricular myocardium when implanted. The basal septal region may include one or more of the basal anteroseptal area 2, basal inferoseptal area 3, mid-anteroseptal area 8, and mid-inferoseptal area 9.

In some embodiments, the tissue-piercing electrode may be positioned in the high inferior/posterior basal septal region of the left ventricular myocardium when implanted. The high inferior/posterior basal septal region of the left ventricular myocardium may include a portion of one or more of the basal inferoseptal area 3 and mid-inferoseptal area 9 (e.g., the basal inferoseptal area only, the mid-inferoseptal area only, or both the basal inferoseptal area and the mid-inferoseptal area). For example, the high inferior/posterior basal septal region may include region 324 illustrated generally as a dashed-line boundary. As shown, the dashed line boundary represents an approximation of where the high inferior/posterior basal septal region is located, which may take a somewhat different shape or size depending on the particular application.

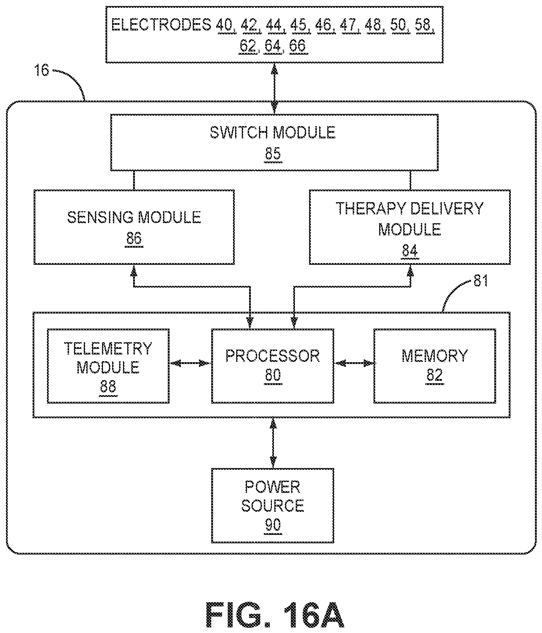

FIG. 16A is a functional block diagram of one illustrative configuration of the IMD 16. As shown, the IMD 16 may include a control module 81, a therapy delivery module 84 (e.g., which may include a stimulation generator), a sensing module 86, and a power source 90.

The control module, or apparatus, 81 may include a processor 80, memory 82, and a telemetry module, or apparatus, 88. The memory 82 may include computer-readable instructions that, when executed, e.g., by the processor 80, cause the IMD 16 and/or the control module 81 to perform various functions attributed to the IMD 16 and/or the control module 81 described herein. Further, the memory 82 may include any volatile, non-volatile, magnetic, optical, and/or electrical media, such as a random-access memory (RAM), read-only memory (ROM), non-volatile RAM (NVRAM), electrically-erasable programmable ROM (EEPROM), flash memory, and/or any other digital media. An illustrative capture management module may be the left ventricular capture management (LVCM) module described in U.S. Pat. No. 7,684,863 entitled "LV THRESHOLD MEASUREMENT AND CAPTURE MANAGEMENT" and issued March 23, 2010.

The processor 80 of the control module 81 may include any one or more of a microprocessor, a controller, a digital signal processor (DSP), an application specific integrated circuit (ASIC), a field-programmable gate array (FPGA), and/or equivalent discrete or integrated logic circuitry. In some examples, the processor 80 may include multiple components, such as any combination of one or more microprocessors, one or more controllers, one or more DSPs, one or more ASICs, and/or one or more FPGAs, as well as other discrete or integrated logic circuitry. The functions attributed to the processor 80 herein may be embodied as software, firmware, hardware, or any combination thereof.

The control module 81 may control the therapy delivery module 84 to deliver therapy (e.g., electrical stimulation therapy such as pacing) to the heart 12 according to a selected one or more therapy programs, which may be stored in the memory 82. More, specifically, the control module 81 (e.g., the processor 80) may control various parameters of the electrical stimulus delivered by the therapy delivery module 84 such as, e.g., A-V delays, V-V delays, pacing pulses with the amplitudes, pulse widths, frequency, or electrode polarities, etc., which may be specified by one or more selected therapy programs (e.g., A-V and/or V-V delay adjustment programs, pacing therapy programs, pacing recovery programs, capture management programs, etc.). As shown, the therapy delivery module 84 is electrically coupled to electrodes 40, 42, 44, 45, 46, 47, 48, 50, 58, 62, 64, 66, e.g., via conductors of the respective lead 18, 20, 22, or, in the case of housing electrode 58, via an electrical conductor disposed within housing 60 of IMD 16. Therapy delivery module 84 may be configured to generate and deliver electrical stimulation therapy such as pacing therapy to the heart 12 using one or more of the electrodes 40, 42, 44, 45, 46, 47, 48, 50, 58, 62, 64, 66.

For example, therapy delivery module 84 may deliver pacing stimulus (e.g., pacing pulses) via ring electrodes 40, 44, 45, 46, 47, 48 coupled to leads 18, 20, 22 and/or helical tip electrodes 42, 50 of leads 18, 22. Further, for example, therapy delivery module 84 may deliver defibrillation shocks to heart 12 via at least two of electrodes 58, 62, 64, 66. In some examples, therapy delivery module 84 may be configured to deliver pacing, cardioversion, or defibrillation stimulation in the form of electrical pulses. In other examples, therapy delivery module 84 may be configured deliver one or more of these types of stimulation in the form of other signals, such as sine waves, square waves, and/or other substantially continuous time signals.

The IMD 16 may further include a switch module 85 and the control module 81 (e.g., the processor 80) may use the switch module 85 to select, e.g., via a data/address bus, which of the available electrodes are used to deliver therapy such as pacing pulses for pacing therapy, or which of the available electrodes are used for sensing. The switch module 85 may include a switch array, switch matrix, multiplexer, or any other type of switching device suitable to selectively couple the sensing module 86 and/or the therapy delivery module 84 to one or more selected electrodes. More specifically, the therapy delivery module 84 may include a plurality of pacing output circuits. Each pacing output circuit of the plurality of pacing output circuits may be selectively coupled, e.g., using the switch module 85, to one or more of the electrodes 40, 42, 44, 45, 46, 47, 48, 50, 58, 62, 64, 66 (e.g., a pair of electrodes for delivery of therapy to a bipolar or multipolar pacing vector). In other words, each electrode can be selectively coupled to one of the pacing output circuits of the therapy delivery module using the switching module 85.

The sensing module 86 is coupled (e.g., electrically coupled) to sensing apparatus, which may include, among additional sensing apparatus, the electrodes 40, 42, 44, 45, 46, 47, 48, 50, 58, 62, 64, 66 to monitor electrical activity of the heart 12, e.g., electrocardiogram (ECG)/electrogram (EGM) signals, etc. The ECG/EGM signals may be used to measure or monitor activation times (e.g., ventricular activations times, etc.), heart rate (HR), heart rate variability (HRV), heart rate turbulence (HRT), deceleration/acceleration capacity, deceleration sequence incidence, T-wave alternans (TWA), P-wave to P-wave intervals (also referred to as the P-P intervals or A-A intervals), R-wave to R-wave intervals (also referred to as the R-R intervals or V-V intervals), P-wave to QRS complex intervals (also referred to as the P-R intervals, A-V intervals, or P-Q intervals), QRS-complex morphology, ST segment (i.e., the segment that connects the QRS complex and the T-wave), T-wave changes, QT intervals, electrical vectors, etc.

The switch module 85 may also be used with the sensing module 86 to select which of the available electrodes are used, or enabled, to, e.g., sense electrical activity of the patient's heart (e.g., one or more electrical vectors of the patient's heart using any combination of the electrodes 40, 42, 44, 45, 46, 47, 48, 50, 58, 62, 64, 66). Likewise, the switch module 85 may also be used with the sensing module 86 to select which of the available electrodes are not to be used (e.g., disabled) to, e.g., sense electrical activity of the patient's heart (e.g., one or more electrical vectors of the patient's heart using any combination of the electrodes 40, 42, 44, 45, 46, 47, 48, 50, 58, 62, 64, 66), etc. In some examples, the control module 81 may select the electrodes that function as sensing electrodes via the switch module within the sensing module 86, e.g., by providing signals via a data/address bus.

In some examples, sensing module 86 includes a channel that includes an amplifier with a relatively wider pass band than the R-wave or P-wave amplifiers. Signals from the selected sensing electrodes may be provided to a multiplexer, and thereafter converted to multi-bit digital signals by an analog-to-digital converter for storage in memory 82, e.g., as an electrogram (EGM). In some examples, the storage of such EGMs in memory 82 may be under the control of a direct memory access circuit.

In some examples, the control module 81 may operate as an interrupt-driven device and may be responsive to interrupts from pacer timing and control module, where the interrupts may correspond to the occurrences of sensed P-waves and R-waves and the generation of cardiac pacing pulses. Any necessary mathematical calculations may be performed by the processor 80 and any updating of the values or intervals controlled by the pacer timing and control module may take place following such interrupts. A portion of memory 82 may be configured as a plurality of recirculating buffers, capable of holding one or more series of measured intervals, which may be analyzed by, e.g., the processor 80 in response to the occurrence of a pace or sense interrupt to determine whether the patient's heart 12 is presently exhibiting atrial or ventricular tachyarrhythmia.

The telemetry module 88 of the control module 81 may include any suitable hardware, firmware, software, or any combination thereof for communicating with another device, such as a programmer. For example, under the control of the processor 80, the telemetry module 88 may receive downlink telemetry from and send uplink telemetry to a programmer with the aid of an antenna, which may be internal and/or external. The processor 80 may provide the data to be uplinked to a programmer and the control signals for the telemetry circuit within the telemetry module 88, e.g., via an address/data bus. In some examples, the telemetry module 88 may provide received data to the processor 80 via a multiplexer.

The various components of the IMD 16 are further coupled to a power source 90, which may include a rechargeable or non-rechargeable battery. A non-rechargeable battery may be selected to last for several years, while a rechargeable battery may be inductively charged from an external device, e.g., on a daily or weekly basis.

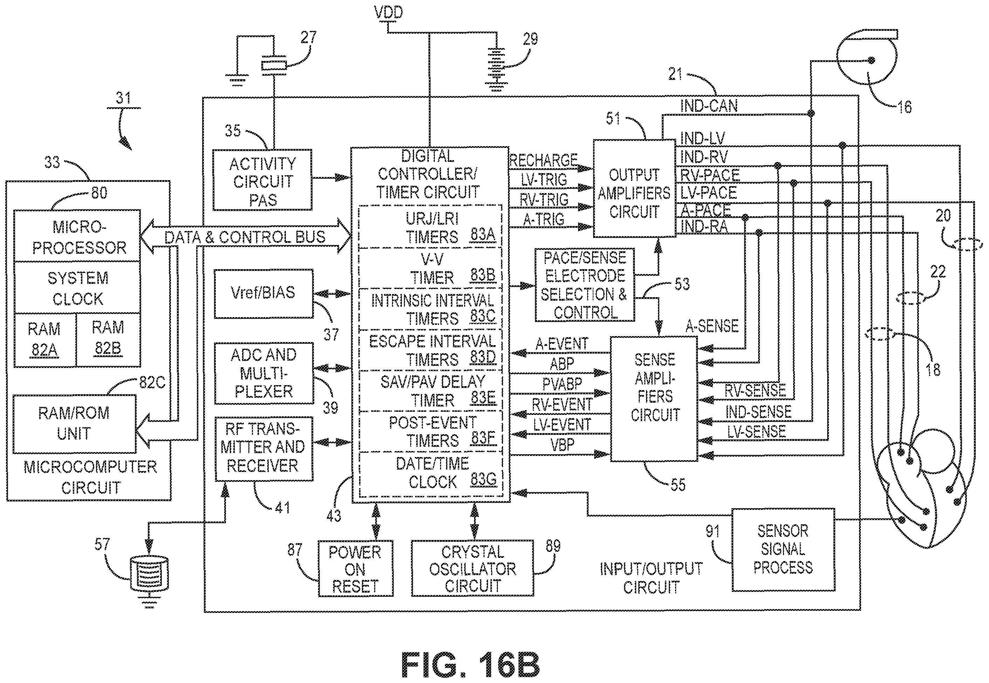

FIG. 16B is another embodiment of a functional block diagram for IMD 16 that depicts bipolar RA lead 22, bipolar RV lead 18, and bipolar LV CS lead 20 without the LA CS pace/sense electrodes and coupled with an implantable pulse generator (IPG) circuit 31 having programmable modes and parameters of a bi-ventricular DDD/R type known in the pacing art. In turn, the sensor signal processing circuit 91 indirectly couples to the timing circuit 43 and via data and control bus to microcomputer circuitry 33. The IPG circuit 31 is illustrated in a functional block diagram divided generally into a microcomputer circuit 33 and a pacing circuit 21. The pacing circuit 21 includes the digital controller/timer circuit 43, the output amplifiers circuit 51, the sense amplifiers circuit 55, the RF telemetry transceiver 41, the activity sensor circuit 35 as well as a number of other circuits and components described below.

Crystal oscillator circuit 89 provides the basic timing clock for the pacing circuit 21 while battery 29 provides power. Power-on-reset circuit 87 responds to initial connection of the circuit to the battery for defining an initial operating condition and similarly, resets the operative state of the device in response to detection of a low battery condition. Reference mode circuit 37 generates stable voltage reference and currents for the analog circuits within the pacing circuit 21. Analog-to-digital converter (ADC) and multiplexer circuit 39 digitize analog signals and voltage to provide, e.g., real time telemetry of cardiac signals from sense amplifiers 55 for uplink transmission via RF transmitter and receiver circuit 41. Voltage reference and bias circuit 37, ADC and multiplexer 39, power-on-reset circuit 87, and crystal oscillator circuit 89 may correspond to any of those used in illustrative implantable cardiac pacemakers.

If the IPG is programmed to a rate responsive mode, the signals output by one or more physiologic sensors are employed as a rate control parameter (RCP) to derive a physiologic escape interval. For example, the escape interval is adjusted proportionally to the patient's activity level developed in the patient activity sensor (PAS) circuit 35 in the depicted, illustrative IPG circuit 31. The patient activity sensor 27 is coupled to the IPG housing and may take the form of a piezoelectric crystal transducer. The output signal of the patient activity sensor 27 may be processed and used as an RCP. Sensor 27 generates electrical signals in response to sensed physical activity that are processed by activity circuit 35 and provided to digital controller/timer circuit 43. Activity circuit 35 and associated sensor 27 may correspond to the circuitry disclosed in U.S. Pat. No. 5,052,388 entitled "METHOD AND APPARATUS FOR IMPLEMENTING ACTIVITY SENSING IN A PULSE GENERATOR" and issued on October 1, 1991 and U.S. Pat. No. 4,428,378 entitled "RATE ADAPTIVE PACER" and issued on January 31, 1984. Similarly, the illustrative systems, apparatus, and methods described herein may be practiced in conjunction with alternate types of sensors such as oxygenation sensors, pressure sensors, pH sensors, and respiration sensors, for use in providing rate responsive pacing capabilities. Alternately, QT time may be used as a rate indicating parameter, in which case no extra sensor is required. Similarly, the illustrative embodiments described herein may also be practiced in non-rate responsive pacemakers.

Data transmission to and from the external programmer is accomplished by way of the telemetry antenna 57 and an associated RF transceiver 41, which serves both to demodulate received downlink telemetry and to transmit uplink telemetry. Uplink telemetry capabilities may include the ability to transmit stored digital information, e.g., operating modes and parameters, EGM histograms, and other events, as well as real time EGMs of atrial and/or ventricular electrical activity and marker channel pulses indicating the occurrence of sensed and paced depolarizations in the atrium and ventricle.

Microcomputer 33 contains a microprocessor 80 and associated system clock and on-processor RAM and ROM chips 82A and 82B, respectively. In addition, microcomputer circuit 33 includes a separate RAM/ROM chip 82C to provide additional memory capacity. Microprocessor 80 normally operates in a reduced power consumption mode and is interrupt driven. Microprocessor 80 is awakened in response to defined interrupt events, which may include A-TRIG, RV-TRIG, LV-TRIG signals generated by timers in digital timer/controller circuit 43 and A-EVENT, RV-EVENT, and LV-EVENT signals generated by sense amplifiers circuit 55, among others. The specific values of the intervals and delays timed out by digital controller/timer circuit 43 are controlled by the microcomputer circuit 33 by way of data and control bus from programmed-in parameter values and operating modes. In addition, if programmed to operate as a rate responsive pacemaker, a timed interrupt, e.g., every cycle or every two seconds, may be provided in order to allow the microprocessor to analyze the activity sensor data and update the basic A-A, V-A, or V-V escape interval, as applicable. In addition, the microprocessor 80 may also serve to define variable, operative A-V delay intervals, V-V delay intervals, and the energy delivered to each ventricle and/or atrium.

In one embodiment, microprocessor 80 is a custom microprocessor adapted to fetch and execute instructions stored in RAM/ROM unit 82 in a conventional manner. It is contemplated, however, that other implementations may be suitable to practice the present disclosure. For example, an off-the-shelf, commercially available microprocessor or microcontroller, or custom application-specific, hardwired logic, or state-machine type circuit may perform the functions of microprocessor 80.

Digital controller/timer circuit 43 operates under the general control of the microcomputer 33 to control timing and other functions within the pacing circuit 21 and includes a set of timing and associated logic circuits of which certain ones pertinent to the present disclosure are depicted. The depicted timing circuits include URI/LRI timers 83A, V-V delay timer 83B, intrinsic interval timers 83C for timing elapsed V-EVENT to V-EVENT intervals or V-EVENT to A-EVENT intervals or the V-V conduction interval, escape interval timers 83D for timing A-A, V-A, and/or V-V pacing escape intervals, an A-V delay interval timer 83E for timing the A-LVp delay (or A-RVp delay) from a preceding A-EVENT or A-TRIG, a post-ventricular timer 83F for timing post-ventricular time periods, and a date/time clock 83G.

The A-V delay interval timer 83E is loaded with an appropriate delay interval for one ventricular chamber (e.g., either an A-RVp delay or an A-LVp) to time-out starting from a preceding A-PACE or A-EVENT. The interval timer 83E triggers pacing stimulus delivery and can be based on one or more prior cardiac cycles (or from a data set empirically derived for a given patient).

The post-event timer 83F times out the post-ventricular time period following an RV-EVENT or LV-EVENT or a RV-TRIG or LV-TRIG and post-atrial time periods following an A-EVENT or A-TRIG. The durations of the post-event time periods may also be selected as programmable parameters stored in the microcomputer 33. The post-ventricular time periods include the PVARP, a post-atrial ventricular blanking period (PAVBP), a ventricular blanking period (VBP), a post-ventricular atrial blanking period (PVARP) and a ventricular refractory period (VRP) although other periods can be suitably defined depending, at least in part, on the operative circuitry employed in the pacing engine. The post-atrial time periods include an atrial refractory period (ARP) during which an A-EVENT is ignored for the purpose of resetting any A-V delay, and an atrial blanking period (ABP) during which atrial sensing is disabled. It should be noted that the starting of the post-atrial time periods and the A-V delays can be commenced substantially simultaneously with the start or end of each A-EVENT or A-TRIG or, in the latter case, upon the end of the A-PACE which may follow the A-TRIG. Similarly, the starting of the post-ventricular time periods and the V-A escape interval can be commenced substantially simultaneously with the start or end of the V-EVENT or V-TRIG or, in the latter case, upon the end of the V-PACE which may follow the V-TRIG. The microprocessor 80 also optionally calculates A-V delays, V-V delays, post-ventricular time periods, and post-atrial time periods that vary with the sensor-based escape interval established in response to the RCP(s) and/or with the intrinsic atrial and/or ventricular rate.

The output amplifiers circuit 51 contains a RA pace pulse generator (and a LA pace pulse generator if LA pacing is provided), a RV pace pulse generator, a LV pace pulse generator, and/or any other pulse generator configured to provide atrial and ventricular pacing. In order to trigger generation of an RV-PACE or LV-PACE pulse, digital controller/timer circuit 43 generates the RV-TRIG signal at the time-out of the A-RVp delay (in the case of RV pre-excitation) or the LV-TRIG at the time-out of the A-LVp delay (in the case of LV pre-excitation) provided by A-V delay interval timer 83E (or the V-V delay timer 83B). Similarly, digital controller/timer circuit 43 generates an RA-TRIG signal that triggers output of an RA-PACE pulse (or an LA-TRIG signal that triggers output of an LA-PACE pulse, if provided) at the end of the V-A escape interval timed by escape interval timers 83D.

The output amplifiers circuit 51 includes switching circuits for coupling selected pace electrode pairs from among the lead conductors and the IND-CAN electrode 20 to the RA pace pulse generator (and LA pace pulse generator if provided), RV pace pulse generator and LV pace pulse generator. Pace/sense electrode pair selection and control circuit 53 selects lead conductors and associated pace electrode pairs to be coupled with the atrial and ventricular output amplifiers within output amplifiers circuit 51 for accomplishing RA, LA, RV and LV pacing.

The sense amplifiers circuit 55 contains sense amplifiers for atrial and ventricular pacing and sensing. High impedance P-wave and R-wave sense amplifiers may be used to amplify a voltage difference signal that is generated across the sense electrode pairs by the passage of cardiac depolarization wavefronts. The high impedance sense amplifiers use high gain to amplify the low amplitude signals and rely on pass band filters, time domain filtering and amplitude threshold comparison to discriminate a P-wave or R-wave from background electrical noise. Digital controller/timer circuit 43 controls sensitivity settings of the atrial and ventricular sense amplifiers 55.

The sense amplifiers may be uncoupled from the sense electrodes during the blanking periods before, during, and after delivery of a pace pulse to any of the pace electrodes of the pacing system to avoid saturation of the sense amplifiers. The sense amplifiers circuit 55 includes blanking circuits for uncoupling the selected pairs of the lead conductors and the IND-CAN electrode 20 from the inputs of the RA sense amplifier (and LA sense amplifier if provided), RV sense amplifier and LV sense amplifier during the ABP, PVABP and VBP. The sense amplifiers circuit 55 also includes switching circuits for coupling selected sense electrode lead conductors and the IND-CAN electrode 20 to the RA sense amplifier (and LA sense amplifier if provided), RV sense amplifier and LV sense amplifier. Again, sense electrode selection and control circuit 53 selects conductors and associated sense electrode pairs to be coupled with the atrial and ventricular sense amplifiers within the output amplifiers circuit 51 and sense amplifiers circuit 55 for accomplishing RA, LA, RV, and LV sensing along desired unipolar and bipolar sensing vectors.

Right atrial depolarizations or P-waves in the RA-SENSE signal that are sensed by the RA sense amplifier result in a RA-EVENT signal that is communicated to the digital controller/timer circuit 43. Similarly, left atrial depolarizations or P-waves in the LA-SENSE signal that are sensed by the LA sense amplifier, if provided, result in a LA-EVENT signal that is communicated to the digital controller/timer circuit 43. Ventricular depolarizations or R-waves in the RV-SENSE signal are sensed by a ventricular sense amplifier result in an RV-EVENT signal that is communicated to the digital controller/timer circuit 43. Similarly, ventricular depolarizations or R-waves in the LV-SENSE signal are sensed by a ventricular sense amplifier result in an LV-EVENT signal that is communicated to the digital controller/timer circuit 43. The RV-EVENT, LV-EVENT, and RA-EVENT, LA-SENSE signals may be refractory or non-refractory and can inadvertently be triggered by electrical noise signals or aberrantly conducted depolarization waves rather than true R-waves or P-waves.

The techniques described in this disclosure, including those attributed to the IMD 16, the computing apparatus 140, and/or various constituent components, may be implemented, at least in part, in hardware, software, firmware, or any combination thereof. For example, various aspects of the techniques may be implemented within one or more processors, including one or more microprocessors, DSPs, ASICs, FPGAs, or any other equivalent integrated or discrete logic circuitry, as well as any combinations of such components, embodied in programmers, such as physician or patient programmers, stimulators, image processing devices, or other devices. The term "module," "processor," or "processing circuitry" may generally refer to any of the foregoing logic circuitry, alone or in combination with other logic circuitry, or any other equivalent circuitry.

Such hardware, software, and/or firmware may be implemented within the same device or within separate devices to support the various operations and functions described in this disclosure. In addition, any of the described units, modules, or components may be implemented together or separately as discrete but interoperable logic devices. Depiction of different features as modules or units is intended to highlight different functional aspects and does not necessarily imply that such modules or units must be realized by separate hardware or software components. Rather, functionality associated with one or more modules or units may be performed by separate hardware or software components or integrated within common or separate hardware or software components.

When implemented in software, the functionality ascribed to the systems, devices and techniques described in this disclosure may be embodied as instructions on a computer-readable medium such as RAM, ROM, NVRAM, EEPROM, FLASH memory, magnetic data storage media, optical data storage media, or the like. The instructions may be executed by processing circuitry and/or one or more processors to support one or more aspects of the functionality described in this disclosure.

## Claims

1. A system (100) for use in cardiac evaluation comprising:
an electrode apparatus (110) comprising a plurality of external electrodes (112) to be disposed proximate a patient's skin; and
a computing apparatus (140) comprising processing circuitry, the computing apparatus operably coupled to the electrode apparatus and configured to:
monitor (410) cardiac electrical activity from tissue of the patient (14) using the plurality of external electrodes;
generate (420) one or more cardiac metrics of the patient based on the monitored electrical activity;
determine (430) whether the patient is a candidate for a cardiac resynchronization therapy device, CRT device, based on a first global dyssynchrony metric using the one or more cardiac metrics if the patient has a right bundle branch block; and
determine (440) whether the patient is a candidate for a cardiac resynchronization therapy device based on a second global dyssynchrony metric using the one or more cardiac metrics if the patient does not have a right bundle branch block.

2. The system of claim 1, wherein the plurality of external electrodes comprise a plurality of left external electrodes positioned to the left side of the patient's torso, wherein the one or more cardiac metrics are more than one cardiac metric comprising a standard deviation of activation times, SDAT, and an average of left ventricular activation times, LVAT, based on the monitored cardiac electrical activity using the plurality of left external electrodes.

3. The system of claim 2, wherein the first global dyssynchrony metric is a sum of the LVAT and a fraction of the SDAT, wherein determining whether the patient is a candidate for a CRT device comprises determining that the patient is a candidate for a CRT device if first global dyssynchrony metric is greater than a predetermined threshold.

4. The system of claim 3, wherein the fraction of the SDAT is (5*SDAT)/3.

5. The system of any of claims 3-4, wherein the predetermined threshold is in a range of about 30 ms to about 80 ms.

6. The system of any of claims 3-5, wherein the predetermined threshold is about 50 ms.

7. The system of any of claims 2-6, wherein the second global dyssynchrony metric comprises:
determining whether the SDAT is greater than a first predetermined threshold; and
determining whether the LVAT is greater than a second predetermined threshold.

8. The system of claim 7, wherein the first predetermined threshold is in a range of about 15 ms to about 30 ms and the second predetermined threshold is in a range of about 25 ms to about 40 ms.

9. The system of any of claims 7-8, wherein the first predetermined threshold is about 25 ms and the second predetermined threshold is about 35 ms.

10. The system of any of claims 3-9, wherein the second global dyssynchrony metric comprises:
determining whether the SDAT divided by a QRSd is greater than a third predetermined threshold; and
determining whether the LVAT divided by the QRSd is greater than a fourth predetermined threshold.

11. The system of claim 10, wherein the third predetermined threshold is in a range of about 0.05 to about 0.25and the fourth predetermined threshold is in a range of about 0.10 to about 0.30.

12. The system of any of claims 10-11, wherein the third predetermined threshold is about 0.15 and the fourth predetermined threshold is about 0.20.

## Patentansprüche

1. System (100) zur Verwendung bei einer kardialen Evaluierung, umfassend:
eine Elektrodeneinrichtung (110), umfassend eine Vielzahl von externen Elektroden (112), die in der Nähe der Haut eines Patienten anzubringen sind; und
eine Recheneinrichtung (140), umfassend eine Verarbeitungsschaltung, wobei die Recheneinrichtung mit der Elektrodeneinrichtung betriebsfähig gekoppelt und konfiguriert ist zum:
Überwachen (410) von kardialer elektrischer Aktivität von dem Gewebe des Patienten (14) unter Verwendung der Vielzahl von externen Elektroden;
Erzeugen (420) einer oder mehrerer kardialer Metriken des Patienten basierend auf der überwachten elektrischen Aktivität;
Bestimmen (430), ob der Patient ein Kandidat für eine kardiale Resynchronisationstherapie-Vorrichtung, CRT-Vorrichtung, ist, basierend auf einer ersten globalen Dyssynchronie-Metrik unter Verwendung der einen oder mehreren kardialen Metriken, falls der Patient einen Rechtsschenkelblock aufweist; und
Bestimmen (440), ob der Patient ein Kandidat für eine kardiale Resynchronisationstherapie-Vorrichtung ist, basierend auf einer zweiten globalen Dyssynchronie-Metrik unter Verwendung der einen oder der mehreren kardialen Metriken, falls der Patient keinen Rechtsschenkelblock aufweist.

2. System nach Anspruch 1, wobei die Vielzahl von externen Elektroden eine Vielzahl von linken externen Elektroden umfasst, die auf der linken Seite des Rumpfes des Patienten positioniert sind, wobei die eine oder mehreren kardialen Metriken mehr als eine kardiale Metrik sind, umfassend eine Standardabweichung von Aktivierungszeiten, SDAT, und einen Durchschnitt von linksventrikulären Aktivierungszeiten, LVAT, basierend auf der überwachten kardialen elektrischen Aktivität unter Verwendung der Vielzahl von linken externen Elektroden.

3. System nach Anspruch 2, wobei die erste globale Dyssynchronie-Metrik eine Summe der LVAT und eines Bruchteils der SDAT ist, wobei das Bestimmen, ob der Patient ein Kandidat für eine CRT-Vorrichtung ist, das Bestimmen umfasst, dass der Patient ein Kandidat für eine CRT-Vorrichtung ist, falls die erste globale Dyssynchronie-Metrik größer als eine vorbestimmte Schwelle ist.

4. System nach Anspruch 3, wobei der Bruchteil der SDAT (5*SDAT)/3 ist.

5. System nach einem der Ansprüche 3 bis 4, wobei die vorbestimmte Schwelle in einem Bereich von etwa 30 ms bis etwa 80 ms liegt.

6. System nach einem der Ansprüche 3 bis 5, wobei die vorbestimmte Schwelle etwa 50 ms beträgt.

7. System nach einem der Ansprüche 2 bis 6, wobei die zweite globale Dyssynchronie-Metrik umfasst:
Bestimmen, ob die SDAT größer als eine erste vorbestimmte Schwelle ist; und
Bestimmen, ob die LVAT größer als eine zweite vorbestimmte Schwelle ist.

8. System nach Anspruch 7, wobei die erste vorbestimmte Schwelle in einem Bereich von etwa 15 ms bis etwa 30 ms liegt und die zweite vorbestimmte Schwelle in einem Bereich von etwa 25 ms bis etwa 40 ms liegt.

9. System nach einem der Ansprüche 7 bis 8, wobei die erste vorbestimmte Schwelle etwa 25 ms beträgt und die zweite vorbestimmte Schwelle etwa 35 ms beträgt.

10. System nach einem der Ansprüche 3 bis 9, wobei die zweite globale Dyssynchronie-Metrik umfasst:
Bestimmen, ob die SDAT dividiert durch eine QRSd größer als eine dritte vorbestimmte Schwelle ist; und
Bestimmen, ob die LVAT dividiert durch die QRSd größer als eine vierte vorbestimmte Schwelle ist.

11. System nach Anspruch 10, wobei die dritte vorbestimmte Schwelle in einem Bereich von etwa 0,05 bis etwa 0,25 liegt und die vierte vorbestimmte Schwelle in einem Bereich von etwa 0,10 bis etwa 0,30 liegt.

12. System nach einem der Ansprüche 10 bis 11, wobei die dritte vorbestimmte Schwelle etwa 0,15 beträgt und die vierte vorbestimmte Schwelle etwa 0,20 beträgt.

## Revendications

1. Système (100) pour utilisation dans une évaluation cardiaque comprenant :
un appareil à électrodes (110) comprenant une pluralité d'électrodes externes (112) devant être disposées à proximité de la peau d'un patient ; et
un appareil informatique (140) comprenant un circuit de traitement, l'appareil informatique étant couplé de manière opérationnelle à l'appareil à électrodes et configuré pour :
surveiller (410) l'activité électrique cardiaque à partir des tissus du patient (14) à l'aide de la pluralité d'électrodes externes ;
générer (420) une ou plusieurs métriques cardiaques du patient en fonction de l'activité électrique surveillée ;
déterminer (430) si le patient est un candidat pour un dispositif de thérapie par resynchronisation cardiaque, dispositif TRC, en fonction d'une première métrique de dyssynchronie globale à l'aide de la ou des métriques cardiaques si le patient a un bloc de branche droit ; et
déterminer (440) si le patient est un candidat pour un dispositif de thérapie par resynchronisation cardiaque en fonction d'une seconde métrique de dyssynchronie globale à l'aide de la ou des métriques cardiaques si le patient n'a pas de bloc de branche droit.

2. Système selon la revendication 1, dans lequel la pluralité d'électrodes externes comprend une pluralité d'électrodes externes gauches positionnées sur le côté gauche du torse du patient, dans lequel la ou les métriques cardiaques sont plus d'une métrique cardiaque comprenant un écart-type des temps d'activation, SDAT, et une moyenne des temps d'activation ventriculaire gauche, LVAT, en fonction de l'activité électrique cardiaque surveillée à l'aide de la pluralité d'électrodes externes gauches.

3. Système selon la revendication 2, dans lequel la première métrique de dyssynchronie globale est une somme du LVAT et d'une fraction du SDAT, dans lequel la détermination si le patient est un candidat pour un dispositif TRC comprend la détermination que le patient est un candidat pour un dispositif TRC si la première métrique de dyssynchronie globale est supérieure à un seuil prédéterminé.

4. Système selon la revendication 3, dans lequel la fraction du SDAT est (5*SDAT)/3.

5. Système selon l'une quelconque des revendications 3-4, dans lequel le seuil prédéterminé est dans une plage d'environ 30 ms à environ 80 ms.

6. Système selon l'une quelconque des revendications 3-5, dans lequel le seuil prédéterminé est d'environ 50 ms.

7. Système selon l'une quelconque des revendications 2-6, dans lequel la seconde métrique de dyssynchronie globale comprend :
la détermination si le SDAT est supérieur à un premier seuil prédéterminé ; et
la détermination si le LVAT est supérieur à un deuxième seuil prédéterminé.

8. Système selon la revendication 7, dans lequel le premier seuil prédéterminé est dans une plage d'environ 15 ms à environ 30 ms et le deuxième seuil prédéterminé est dans une plage d'environ 25 ms à environ 40 ms.

9. Système selon l'une quelconque des revendications 7-8, dans lequel le premier seuil prédéterminé est d'environ 25 ms et le deuxième seuil prédéterminé est d'environ 35 ms.

10. Système selon l'une quelconque des revendications 3-9, dans lequel la seconde métrique de dyssynchronie globale comprend :
la détermination si le SDAT divisé par un QRSd est supérieur à un troisième seuil prédéterminé ; et
déterminer si le LVAT divisé par le QRSd est supérieur à un quatrième seuil prédéterminé.

11. Système selon la revendication 10, dans lequel le troisième seuil prédéterminé est dans une plage d'environ 0,05 à environ 0,25 et le quatrième seuil prédéterminé est dans une plage d'environ 0,10 à environ 0,30.

12. Système selon l'une quelconque des revendications 10-11, dans lequel le troisième seuil prédéterminé est d'environ 0,15 et le quatrième seuil prédéterminé est d'environ 0,20.
